(19) Europäisches Patentamt
European Patent Office
Office européen des brevets

(11) EP 4 785 939 A1

(12) **EUROPEAN PATENT APPLICATION**
published in accordance with Art. 153(4) EPC

(43) Date of publication:
**05.08.2026 Bulletin 2026/32**

(21) Application number: **24877223.8**

(22) Date of filing: **10.10.2024**

(51) International Patent Classification (IPC):
***A61K 31/405*** *(2006.01)* ***A61P 3/00*** *(2006.01)*
***A61P 27/16*** *(2006.01)* ***A61P 43/00*** *(2006.01)*

(52) Cooperative Patent Classification (CPC):
**A61K 31/405; A61P 3/00; A61P 27/16; A61P 43/00**

(86) International application number:
**PCT/JP2024/036222**

(87) International publication number:
**WO 2025/079629 (17.04.2025 Gazette 2025/16)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **11.10.2023 PCT/JP2023/036841**

(71) Applicant: **Tohoku University**
**Sendai-shi, Miyagi 980-8577 (JP)**

(72) Inventors:
- **ABE Takaaki**
  **Sendai-shi, Miyagi 980-8577 (JP)**
- **MATSUMOTO Yotaro**
  **Sendai-shi, Miyagi 980-8577 (JP)**
- **KUJIRAI Ryota**
  **Sendai-shi, Miyagi 980-8577 (JP)**
- **SUZUKI Takehiro**
  **Sendai-shi, Miyagi 980-8577 (JP)**
- **HIGASHITANI Atsushi**
  **Sendai-shi, Miyagi 980-8577 (JP)**
- **SATO Takeya**
  **Sendai-shi, Miyagi 980-8577 (JP)**
- **KWON Eunsang**
  **Sendai-shi, Miyagi 980-8577 (JP)**

(74) Representative: **Hoffmann Eitle**
**Patent- und Rechtsanwälte PartmbB**
**Arabellastraße 30**
**81925 München (DE)**

# (54) S(+) FORM OF 4-(2,4-DIFLUOROPHENYL)-2-(1H-INDOL-3-YL)-4-OXOBUTANOIC ACID

(57) The present invention relates to use of the S(+) form of 4-(2,4-difluorophenyl)-2-(1H-indol-3-yl)-4-oxobutanoic acid or a physiologically acceptable salt thereof as an active ingredient of a pharmaceutical composition or a food or drink composition, and the like.

EP 4 785 939 A1

## Description

## Technical Field

Related Application:

The present specification includes the contents described in the specification of PCT/JP2023/036841 (filed on October 11, 2023), which is the basis of priority of the present application.

Technical Field:

The present invention relates to use of the enantiomer S(+) form of 4-(2,4-difluorophenyl)-2-(1H-indol-3-yl)-4-oxobutanoic acid (hereinafter sometimes referred to as "MA5") or a physiologically acceptable salt thereof as an active ingredient of a pharmaceutical composition or a food or drink composition, and the like.

## Background Art

The present inventors have reported that the racemic form of 4-(2,4-difluorophenyl)-2-(1H-indol-3-yl)-4-oxobutanoic acid (that is, MA5) has an erythropoietin expression enhancing effect and a therapeutic effect on a mitochondrial disease (Patent Literature 1), an organ fibrosis suppressing effect (Patent Literature 2), and a preventive or improving effect on hearing loss (Patent Literature 3). Furthermore, the present inventors have reported that the enantiomer R(-) form of MA5, has the action of increasing the amount of oxidized NAD; nicotinamide adenine dinucleotide ($NAD^+$) in an animal cell, and the like (Patent Literature 4).

## Citation List

### Patent Literature

[Patent Literature 1] International Publication No. WO 2014/080640
[Patent Literature 2] Japanese Unexamined Patent Publication No. 2015-189670
[Patent Literature 3] Japanese Unexamined Patent Publication No. 2019-116453
[Patent Literature 4] International Publication No. WO 2023/013756

## Summary of Invention

The present inventors made each enantiomer of MA5 into a benzylamine salt, which is easy to crystallize, carried out X-ray crystallography thereof, and realized that there was an error in the absolute configuration disclosed in a previous report. That is, the present inventors have confirmed that it is the S(+) form of MA5, not the R(-) form thereof, that has the action of increasing the amount of $NAD^+$ in an animal cell and has mitochondrial function activating and anti-aging actions.
The present invention relates to a pharmaceutical composition, a food or drink composition, or the like including the S(+) form of MA5 as an active ingredient, and provides the following [1] to [17].

[1] A pharmaceutical composition comprising a compound represented by the following formula (A-1), substantially free of an R(-) form, or a physiologically acceptable salt thereof as an active ingredient.

[Chemical Formula 1]

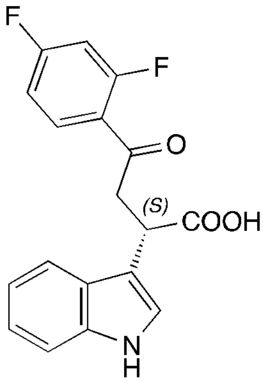

(A-1)

[2] The pharmaceutical composition according to [1], wherein the pharmaceutical composition is used to increase an $NAD^+$ level in a subject.
[3] The pharmaceutical composition according to [1] or [2], wherein the pharmaceutical composition is for treatment or prevention of a mitochondrial disease.
[4] The pharmaceutical composition according to [1] or [2], wherein the pharmaceutical composition is for treatment or prevention of a symptom or a disease associated with aging.
[5] The pharmaceutical composition according to [4], wherein the symptom or the disease associated with aging is hearing loss.
[6] The pharmaceutical composition according to any one of [1] to [5], wherein an enantiomeric excess of an S(+) form is at least 97%.
[7] The pharmaceutical composition according to any of [1] to [6], wherein the compound represented by formula (A-1) or the physiologically acceptable salt thereof is a sodium salt, a potassium salt, or a calcium salt, preferably a sodium salt or a potassium salt, and more preferably a sodium salt, of the compound represented by formula (A-1).
[8] A food or drink composition comprising a compound represented by the following formula (A-1), substantially free of an R(-) form, or a physiologically acceptable salt thereof as an active ingredient.

[Chemical Formula 2]

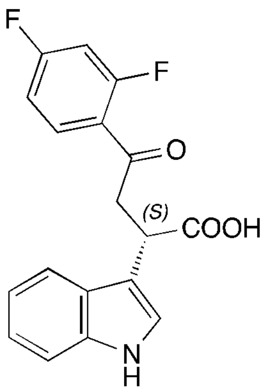

(A-1)

[9] The food or drink composition according to [8], wherein the food or drink composition is used to increase an $NAD^+$ level in a subject.
[10] The food or drink composition according to [8] or [9], wherein the food or drink composition is for a subject in need of treating or preventing a mitochondrial disease.
[11] The food or drink composition according to [8] or [9], wherein the food or drink composition is for a subject in need of treating or preventing a symptom or a disease associated with aging.
[12] The food or drink composition according to [11], wherein the symptom or the disease associated with aging is hearing loss.
[13] The food or drink composition according to any one of [8] to [12], wherein an enantiomeric excess of an S(+) form is at least 97%.

[14] The food or drink composition according to any of [8] to [13], wherein the compound represented by formula (A-1) or the physiologically acceptable salt thereof is a sodium salt, a potassium salt, or a calcium salt, preferably a sodium salt or a potassium salt, and more preferably a sodium salt, of the compound represented by formula (A-1).

[15] The pharmaceutical composition according to any one of [1] to [7], or the food or drink composition according to any one of [8] to [14], wherein the pharmaceutical composition or the food or drink composition is stored with protection from light.

[16] The pharmaceutical composition according to any one of [1] to [7], or the food or drink composition according to any one of [8] to [14], wherein the pharmaceutical composition or the food or drink composition is stored near neutrality, preferably at pH 6.0 to 8.0.

[17] The pharmaceutical composition according to any of [1] to [7], or the food or drink composition according to any of [8] to [14], wherein when the pharmaceutical composition or the food or drink composition comprises chloroform, a content of a short chain alcohol based on chloroform is less than 1%, and the pharmaceutical composition or the food or drink composition preferably does not comprise the short chain alcohol and chloroform at the same time (the pharmaceutical composition or the food or drink composition comprises chloroform and no short chain alcohol; comprises the short chain alcohol and no chloroform; or comprises neither a short chain alcohol nor chloroform).

In another embodiment, the present invention provides the following [1] to [17] (the compound represented by formula (A-1) is as described above).

[1] A compound represented by formula (A-1), substantially free of an R(-) form, for use in treatment or prevention of a subject in need of increasing an $NAD^+$ level, or for use in a method for increasing an $NAD^+$ level in a subject, or a physiologically acceptable salt thereof.

[2] A compound represented by formula (A-1), substantially free of an R(-) form, for use in treatment or prevention of a mitochondrial disease, or a physiologically acceptable salt thereof.

[3] A compound represented by formula (A-1) for use in treatment or prevention of a symptom or a disease associated with aging, or a physiologically acceptable salt thereof.

[4] The compound for use according to [3], wherein the symptom or the disease associated with aging is any of the group consisting of hearing loss (more specifically, age-related hearing loss), visual impairment (more specifically, age-related visual impairment), anemia (more specifically, age-related anemia), muscle weakness (more specifically, age-related muscle weakness), thinning hair or hair loss (more specifically, age-related thinning hair or hair loss), and shortened lifespan (more specifically, age-related shortened lifespan) and is preferably hearing loss, or a physiologically acceptable salt thereof.

[5] The compound for use according to any one of [2] to [4], wherein the treatment or the prevention is provided by increasing an $NAD^+$ level in a subject, or a physiologically acceptable salt thereof.

[6] The compound for use according to any one of [1] to [5], wherein an enantiomeric excess of an S(+) form is at least 97%, preferably 97.5% or more, more preferably 98% or more, particularly preferably 98.5% or more, and further preferably 99% or more, for example 99.5%, 99.6%, 99.7%, 99.8%, or 99.9% or more, or a physiologically acceptable salt thereof.

[7] The compound for use according to any of [1] to [6] or the physiologically acceptable salt thereof, wherein the compound represented by formula (A-1) or the physiologically acceptable salt thereof is a sodium salt, a potassium salt, or a calcium salt, preferably a sodium salt or a potassium salt, and more preferably a sodium salt, of the compound represented by formula (A-1).

[8] A non-therapeutic use of a compound represented by formula (A-1), substantially free of an R(-) form, or a physiologically acceptable salt thereof, wherein the compound represented by formula (A-1) or the physiologically acceptable salt thereof is used to increase an $NAD^+$ level.

[9] A non-therapeutic use of a compound represented by formula (A-1), substantially free of an R(-) form, or a physiologically acceptable salt thereof, wherein the compound represented by formula (A-1) or the physiologically acceptable salt thereof is used to improve or prevent a mitochondrial disease.

[10] A non-therapeutic use of a compound represented by formula (A-1), substantially free of an R(-) form, or a physiologically acceptable salt thereof, wherein the compound represented by formula (A-1) or the physiologically acceptable salt thereof is used to improve or prevent a symptom or a disease associated with aging.

[11] The non-therapeutic use according to [10], wherein the symptom or the disease associated with aging is any of the group consisting of hearing loss (more specifically, age-related hearing loss), visual impairment (more specifically, age-related visual impairment), anemia (more specifically, age-related anemia), muscle weakness (more specifically, age-related muscle weakness), thinning hair or hair loss (more specifically, age-related thinning hair or hair loss), and shortened lifespan (more specifically, age-related shortened lifespan) and is preferably hearing loss.

[12] The non-therapeutic use according to any one of [9] to [11], wherein the improvement or the prevention is provided by increasing an $NAD^+$ level in a subject.

[13] The non-therapeutic use according to any one of [8] to [12], wherein in the compound represented by formula (A-1) or the physiologically acceptable salt thereof, an enantiomeric excess of an S(+) form is at least 97.5%, more preferably 98% or more, particularly preferably 98.5% or more, and further preferably 99% or more, for example 99.5%, 99.6%, 99.7%, 99.8%, or 99.9% or more.

[14] The non-therapeutic use according to any of [8] to [13], wherein the compound represented by formula (A-1) or the physiologically acceptable salt thereof is a sodium salt, a potassium salt, or a calcium salt, preferably a sodium salt or a potassium salt, and more preferably a sodium salt, of the compound represented by formula (A-1).

[15] The compound for use according to any one of [1] to [7], or a physiologically acceptable salt thereof, or the non-therapeutic use according to any one of [8] to [14], wherein the compound represented by formula (A-1) or the physiologically acceptable salt thereof is present in a formulation stored with protection from light.

[16] The compound for use according to any one of [1] to [7], or a physiologically acceptable salt thereof, or the non-therapeutic use according to any one of [8] to [14], wherein the compound represented by formula (A-1) or the physiologically acceptable salt thereof is present in a formulation near neutrality, preferably at pH 6.0 to 8.0.

[17] The compound for use according to any one of [1] to [7], or a physiologically acceptable salt thereof, or the non-therapeutic use according to any one of [8] to [14], wherein when the compound represented by formula (A-1) or the physiologically acceptable salt thereof is present in a formulation comprising chloroform, a content of a short chain alcohol based on chloroform is less than 1%, and preferably the compound represented by formula (A-1) or the physiologically acceptable salt thereof is present in a formulation that does not comprise a short chain alcohol and chloroform at the same time. The phrase "not comprise ... at the same time" means comprising chloroform and no short chain alcohol, comprising the short chain alcohol and no chloroform, or comprising neither a short chain alcohol nor chloroform.

In another embodiment, the present invention provides the following [1] to [8] (the compound represented by formula (A-1) is as described above).

[1] A method for increasing an $NAD^+$ level in a subject, comprising administering a compound represented by formula (A-1), substantially free of an R(-) form, or a physiologically acceptable salt thereof to the subject in need thereof.

[2] A method for treating, improving, or preventing a mitochondrial disease, comprising administering a compound represented by formula (A-1), substantially free of an R(-) form, or a physiologically acceptable salt thereof to a subject in need thereof.

[3] A method for treating, improving, or preventing a symptom or a disease associated with aging, comprising administering a compound represented by formula (A-1), substantially free of an R(-) form, or a physiologically acceptable salt thereof to a subject in need thereof.

[4] The method according to [3], wherein the symptom or the disease associated with aging is any selected from the group consisting of visual impairment, anemia, muscle weakness, thinning hair or hair loss, and shortened lifespan.

[5] The method according to [3], wherein the symptom or the disease associated with aging is hearing loss.

[6] The method according to [1], wherein the compound represented by formula (A-1) or the physiologically acceptable salt thereof is a sodium salt, a potassium salt, or a calcium salt, preferably a sodium salt or a potassium salt, and more preferably a sodium salt, of the compound represented by formula (A-1).

[7] The method according to [2], wherein the compound represented by formula (A-1) or the physiologically acceptable salt thereof is a sodium salt, a potassium salt, or a calcium salt, preferably a sodium salt or a potassium salt, and more preferably a sodium salt, of the compound represented by formula (A-1).

[8] The method according to [3], wherein the compound represented by formula (A-1) or the physiologically acceptable salt thereof is a sodium salt, a potassium salt, or a calcium salt, preferably a sodium salt or a potassium salt, and more preferably a sodium salt, of the compound represented by formula (A-1).

In addition, other embodiments of the present invention include: use of an S(+) form of MA5 or a physiologically acceptable salt thereof in production of a therapeutic agent or a preventive agent for a mitochondrial disease;

use of an S(+) form of MA5 or a physiologically acceptable salt thereof in production of a therapeutic agent or a preventive agent for a symptom or a disease associated with aging;

a method for promoting generation of $NAD^+$ in an animal (subject) cell, comprising a step of administering an S(+) form of MA5 or a physiologically acceptable salt thereof to a subject in need of promoting generation of $NAD^+$ in an animal cell;

a method for in vitro promoting generation of $NAD^+$ in an animal cell, comprising a step of culturing an animal cell in the presence of an S(+) form of MA5 or a physiologically acceptable salt thereof (hereinafter sometimes referred to as "the present promotion method");

a mitochondrial function activating agent in an animal, comprising an S(+) form of MA5 or a physiologically acceptable

salt thereof;

a method for activating a mitochondrial function in an animal, comprising a step of administering an S(+) form of MA5 or a physiologically acceptable salt thereof to a subject in need of activating the mitochondrial function;

an agent for improving motor dysfunction in an animal, comprising an S(+) form of MA5 or a physiologically acceptable salt thereof; and

a method for improving motor dysfunction in an animal, comprising a step of administering an S(+) form of MA5 or a physiologically acceptable salt thereof to a subject in need of improving the motor dysfunction. In the above-mentioned embodiment, the compound represented by formula (A-1) or the physiologically acceptable salt thereof is preferably a sodium salt, a potassium salt, or a calcium salt, more preferably a sodium salt or a potassium salt, and more preferably a sodium salt, of the compound represented by formula (A-1).

## Advantageous Effects of Invention

Unlike the racemic form of MA5 and the R(-) form of MA5, the S(+) form of MA5, which is a compound of the present invention, has the action of increasing the amount of $NAD^+$ in an animal cell. In addition, unlike the R(-) form of MA5, the S(+) form of MA5 has the action of increasing the expression level of an SIRT in an animal cell. Furthermore, the S(+) form of MA5 is less likely to be metabolized into a toxic substance (glucuronide conjugate) in vivo, is more likely to be retained in blood, and has lower cytotoxicity to an animal cell than the R(-) form of MA5.

On the other hand, it is known that the $NAD^+$ level in an animal cell decreases with age and plays an important role in the onset of an age-related disease. In addition, it has been reported that increasing the amount of $NAD^+$ extends the lifespan in C. elegans and provides a protective action against a metabolic disease in a mouse (see the literature "Nature. 2018 563(7731) 354-359."). In addition, C. elegans is used as a model organism for human aging (see the literature "Bulletin of the Institute of Advanced Biosciences, Tokai University, Volume 1, March 2017").

Because of these, when the S(+) form of MA5 is administered to an animal, it increases the amount of $NAD^+$ in an animal cell without causing any adverse effect such as a side effect, and as a result, it can be fully expected that the lifespan can be extended, or a symptom or a disease such as a symptom or a disease associated with aging or a metabolic disease can be treated or prevented.

Furthermore, the S(+) form of MA5 has the action of improving a mitochondrial function in a cell, and thus is useful as a mitochondrial function activating agent or a therapeutic agent or a preventive agent for a mitochondrial disease.

## Brief Description of Drawings

[Figure 1] Figure 1 is a diagram showing results obtained by culturing a rat pancreatic β cell line (INS-1E cell line) for 6 hours in the presence of 0.1% DMSO ("0" in the figure) or various concentrations (3 pM, 10 pM, 30 pM, 100 pM, 300 pM, 1 nM, or 3 nM) of three compounds (racemic form of MA5 [Figure 1A], S(+) form of MA5 [Figure 1B], or R(-) form of MA5 [Figure 1C]), and measuring the amount of $NAD^+$ in cells (average value ± standard deviation [SE]). "*" and "**" in the figure indicate a statistically significant increase ($p < 0.05$ and $p < 0.01$, respectively) as compared with the results in the absence of the compounds, and "#" and "##" in the figure indicate a statistically significant decrease ($p < 0.05$ and $p < 0.01$, respectively) as compared with the results with 0.1% DMSO.

[Figure 2] Figure 2 is a diagram showing results obtained by incubating human liver microsomes in the presence of three compounds (racemic form of MA5 ["racemi" in the figure], S(+) form of MA5 ["S" in the figure], or R(-) form of MA5 ["R" in the figure]) or in the absence of these compounds ("Control" in the figure) and analyzing the generated metabolites by mass spectrometry.

[Figure 3] Figure 3 is a diagram showing results obtained by culturing a human bladder epithelial-derived cell line (HBlEpC cell line) in the presence of 0.1% DMSO ("0" in the figure) or various concentrations (30 nM, 100 nM, 300 nM, 1 μM, 3 μM, 10 μM, 30 μM, 100 μM, 300 μM, or 1 mM) of three compounds (racemic form of MA5 [Figure 3A], S(+) form of MA5 [Figure 3B], or R(-) form of MA5 [Figure 3C]) and analyzing the viable cell level (average value ± SE). The "viable cell level" on the ordinate indicates the value of optical density ($OD_{450}$) at a wavelength of 450 nm measured by a WST-8 assay. "*" and "* *" in the figure indicate that there is a statistically significant difference ($p < 0.05$ and $p < 0.01$, respectively) as compared with the results with 0.1% DMSO.

[Figure 4] Figure 4 is a diagram showing results obtained by culturing a fibroblast cell line derived from a Leigh encephalopathy patient (MT-ND3 mutant cell line) in the presence of 0.1% DMSO ("0" in the figure) or various concentrations (0.003 μM, 0.01 μM, 0.03 μM, 0.1 μM, 0.3 μM, 1 μM, 3 μM, 10 μM, 30 μM, or 100 μM) of each enantiomer of MA5 (S (+) form ["S" in the figure] or R(-) form ["R" in the figure]) and measuring the viable cell level (average value ± SE). "*" in the figure indicates that there is a statistically significant difference ($p < 0.05$) as compared with the results with 0.1% DMSO.

[Figure 5] Figure 5 is a diagram showing results obtained by culturing the MT-ND3 mutant cell line in the presence of 0.1% DMSO ("0" in the figure) or various concentrations (0.1 μM or 1 μM) of three compounds (racemic form of MA5 ["racemi" in the figure], S(+) form of MA5 ["S" in the figure], or R(-) form of MA5 ["R" in the figure]) and measuring the amount of ATP produced in the culture medium. "*" in the figure indicates that there is a statistically significant difference ($p < 0.05$) as compared with the results with 0.1% DMSO.

[Figure 6] Figure 6 is fluorescence images of mitochondria in body wall muscle cells of ccIs4251 wild-type C. elegans ("WT" in the figure; n = 18) or immt-1/mitofilin mutants ("immt-1/mitofilin mutant mock"; n = 41, "immt-1/mitofilin mutant MA-5 (racemi)"; n = 37, "immt-1/mitofilin mutant MA-5S"; n = 61, and "immt-1/mitofilin mutant MA-5R"; n = 34) raised in the presence of four substances (DMSO or 10 μM racemic form of MA5, S(+) form of MA5, or R(-) form of MA5, respectively).

[Figure 7] Figure 7 is a diagram showing results obtained by analyzing mitochondrial swelling levels in body wall muscle cells based on the fluorescence images in Figure 6.

[Figure 8] Figure 8 is a diagram showing results obtained by analyzing mitochondrial swelling levels in body wall muscle cells based on the fluorescence images in Figure 6. It is indicated that there is a statistically significant difference ($p < 0.05$) between "a" and "b" in the figure. "×" in the figure indicates an average value, and "○" in the figure indicates an outlier.

[Figure 9] Figure 9 is a diagram showing results obtained by orally administering the S(+) form of MA5 and the R(-) form of MA5 to cynomolgus monkeys, respectively, and measuring the concentrations of the S(+) form of MA5 and the R(-) form of MA5 in the plasma after administration.

[Figure 10] Figure 10 is a diagram showing results obtained by orally administering the S(+) form of MA5 and the R(-) form of MA5 to cynomolgus monkeys, respectively, and measuring the concentrations of a reduced metabolite of MA5 (compound represented by formula (B) [hereinafter also referred to as "Red-MA5"]) in the plasma after administration.

[Figure 11] Figure 11 is a diagram showing results obtained by culturing a fibroblast cell line derived from a Leigh encephalopathy patient (KCMC10 cell line) in the presence of 0.1% DMSO ("0" in the figure) or various concentrations (3 nM, 10 nM, 30 nM, 100 nM, 300 nM, 1 μM, 3 μM, 10 μM, or 30 μM) of two compounds (racemic form of MA5 [Figure 11A] or Red-MA5 [Figure 11B]) and measuring the viable cell level (average value ± SE). "*" in the figure indicates that there is a statistically significant difference ($p < 0.05$) as compared with the results with 0.1% DMSO.

[Figure 12-1] Figure 12-1 is a diagram showing results obtained by culturing a mouse inner ear cell line (HEI-OC1 cell line) in the presence of 0.1% DMSO or various concentrations (1 μM, 3 μM, 10 μM, or 30 μM) of the racemic form of MA5, and analyzing the expression levels of various SIRTs (SIRT1, SIRT2 isoform 1, SIRT2 isoform 2, and SIRT3) in the cells by Western blotting. The lower graphs show results obtained by expressing each band intensity in the upper results as a relative value when the band intensity in 0.1% DMSO, which is the control, is set to 1.

[Figure 12-2] Figure 12-2 is a diagram showing results obtained by culturing the HEI-OC1 cell line in the presence of 0.1% DMSO or various concentrations (1 μM, 3 μM, 10 μM, or 30 μM) of the racemic form of MA5, and analyzing the expression levels of various SIRTs (SIRT5, SIRT6, and SIRT7) and the expression level of β-actin (Figure 13D) in the cells by Western blotting. The lower graphs show results obtained by expressing each band intensity in the upper results as a relative value when the band intensity in 0.1% DMSO, which is the control, is set to 1.

[Figure 13-1] Figure 13-1 is a diagram showing results obtained by culturing the HEI-OC1 cell line in the presence of 0.1% DMSO or various concentrations (1 μM, 3 μM, 10 μM, or 30 μM) of the S(+) form of MA5, and analyzing the expression levels of various SIRTs (SIRT1, SIRT2 isoform 1, SIRT2 isoform 2, and SIRT3) in the cells by Western blotting. The lower graphs show results obtained by expressing each band intensity in the upper results as a relative value when the band intensity in 0.1% DMSO, which is the control, is set to 1.

[Figure 13-2] Figure 13-2 is a diagram showing results obtained by culturing the HEI-OC1 cell line in the presence of 0.1% DMSO or various concentrations (1 μM, 3 μM, 10 μM, or 30 μM) of the S(+) form of MA5, and analyzing the expression levels of various SIRTs (SIRT5, SIRT6, and SIRT7) and the expression level of β-actin in the cells by Western blotting. The lower graphs show results obtained by expressing each band intensity in the upper results as a relative value when the band intensity in 0.1% DMSO, which is the control, is set to 1.

[Figure 14-1] Figure 14-1 is a diagram showing results obtained by culturing the HEI-OC1 cell line in the presence of 0.1% DMSO or various concentrations (1 μM, 3 μM, 10 μM, or 30 μM) of the R(-) form of MA5, and analyzing the expression levels of various SIRTs (SIRT1, SIRT2 isoform 1, SIRT2 isoform 2, and SIRT3) in the cells by Western blotting. The lower graphs show results obtained by expressing each band intensity in the upper results as a relative value when the band intensity in 0.1% DMSO, which is the control, is set to 1.

[Figure 14-2] Figure 14-2 is a diagram showing results obtained by culturing the HEI-OC1 cell line in the presence of 0.1% DMSO or various concentrations (1 μM, 3 μM, 10 μM, or 30 μM) of the R(-) form of MA5, and analyzing the expression levels of various SIRTs (SIRT5, SIRT6, and SIRT7) and the expression level of β-actin in the cells by Western blotting. The lower graphs show results obtained by expressing each band intensity in the upper results as a relative value when the band intensity in 0.1% DMSO, which is the control, is set to 1.

[Figure 15] Figure 15 shows results of a test of binding of the S(+) form and the R(-) form of MA5 to NAMPT. The Kd

value of the S(+) form of MA5 was 58.94 μM, whereas the R(-) form of MA5 did not bind to NAMPT, and the Kd value was unable to be measured.

[Figure 16] Figure 16 shows the fluorescence intensity by immunostaining of γH2AX (phosphorylated H2AX). Only when the S(+) form of MA5 was added, the level of yH2AX was low, and a suppressing action on DNA damage was observed.

[Figure 17] Figure 17 shows chromatograms of MA5 using a preparative separation apparatus (LC-Forte). As a result of X-ray crystallography of the corresponding benzylamine salts, it was determined that the absolute configuration of MA5 in the pre-elution fraction was S, and that the absolute configuration of MA5 in the post-elution fraction was R. In addition, as a result of measuring the optical rotation of MA5, it was determined that MA5 in the pre-elution fraction was dextrorotatory (+), and that MA5 in the post-elution fraction was levorotatory (-).

**Description of Embodiments**

The compound of the present invention is the compound represented by the following formula (A-1) (that is, the enantiomer S(+) form of the compound represented by the following formula (A) (4-(2,4-difluorophenyl)-2-(1H-indol-3-yl)-4-oxobutanoic acid (MA5)) or a physiologically acceptable salt thereof.

[Chemical Formula 3]

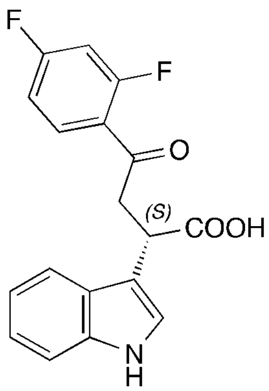

(A-1)

[Chemical Formula 4]

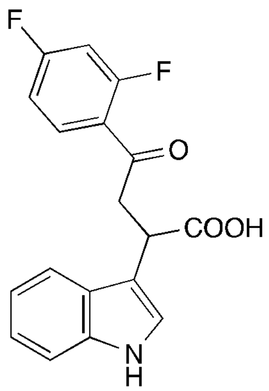

(A)

The S(+) form of MA5 used in the present invention is substantially free of the R(-) form of MA5. "Substantially free of the R(-) form of MA5" means that the enantiomeric excess of the S(+) form is about 97% or more, preferably about 97.5% or more, more preferably about 98% or more, particularly preferably about 98.5% or more, and further preferably about 99% or more, for example, about 99.5%, 99.6%, 99.7%, 99.8%, or 99.9% or more.

The "enantiomeric excess" (also written as "ee") refers to the excess of one enantiomer over the other enantiomer, and when the S form is in excess, the enantiomeric excess is expressed by the following expression.

$$ee = (A_S - A_R)/(A_S + A_R)$$

$A_R$ and $A_S$ represent the molar fractions of the R form and the S form, respectively.

As used herein, "about" refers to a value that may vary up to plus or minus 10%, 8%, 6%, 5%, 4%, 3%, 2%, or 1% from the reference value. Preferably, the term "about" refers to a range of plus or minus 10%, 5%, or 1% from the reference value.

As used herein, "physiologically acceptable salt" is a salt that, within the scope of an appropriate medical, pharmaceutical, or biological determination, is for use in contact with an animal tissue without causing undue toxicity, irritation, allergic response, or other problem or complication, and commensurate with a moderate benefit-risk ratio. Examples of the physiologically acceptable salt include an ammonium salt; an alkali metal salt such as a sodium salt, a lithium salt, or a potassium salt; an alkaline earth metal salt such as an aluminum salt, a calcium salt, or a magnesium salt; a salt with an organic base such as a dicyclohexylamine salt or N-methyl-D-glucamine; a salt with an amino acid such as arginine, lysine, or ornithine; and a salt generated by a basic nitrogen-containing group.

The S(+) form of MA5, represented by formula (A-1), or the salt thereof is preferably a sodium salt, a potassium salt, or a calcium salt, more preferably a sodium salt or a potassium salt, and further preferably a sodium salt, of the S(+) form of MA5. This is because, as shown in Examples, described below, it has been confirmed that if the S(+) form of MA5 is formed into a salt such as a sodium salt, a potassium salt, or a calcium salt, the water solubility is improved; in particular, the sodium salt or the potassium salt exhibit excellent water solubility, and the sodium salt further exhibits excellent photostability.

The S(+) form of MA5, represented by formula (A-1), in the present invention can be obtained according to an organic synthesis technique using a known organic chemical reaction, for example, the method described in the section "1. Generation of S(+) form of MA5" in the Examples described below.

The present invention provides a pharmaceutical composition or a food or drink composition including the S(+) form of MA5 or a physiologically acceptable salt thereof as an active ingredient. The pharmaceutical composition or the food or drink composition is substantially free of the R(-) form of MA5 or a physiologically acceptable salt thereof. "Substantially free of the R(-) form of MA5 or a physiologically acceptable salt thereof" means that the enantiomeric excess of the S(+) form is about 97% or more, preferably about 97.5% or more, more preferably about 98% or more, particularly preferably about 98.5% or more, and further preferably about 99% or more, for example, about 99.5%, 99.6%, 99.7%, 99.8%, or 99.9% or more.

In one embodiment, the pharmaceutical composition or the food or drink composition is used to promote generation of $NAD^+$ in an animal cell, and for example, to increase the $NAD^+$ level in a subject in need thereof.

In another embodiment, the pharmaceutical composition or the food or drink composition is used to activate a mitochondrial function in a subject, and for example, to treat or prevent a mitochondrial disease in a subject in need thereof.

In another embodiment, the pharmaceutical composition or the food or drink composition is used to treat or prevent a symptom or a disease associated with aging in a subject in need thereof.

Examples of the food or drink composition include a health food (functional food, nutritional supplement, dietary supplement, fortified food, nutrient controlling food, supplement, or the like) and a food with health claims (food for specified health uses, food with nutrient function claims, food with function claims, or the like).

Herein, examples of the animal include a mammal (human or non-human mammal), a bird, a reptile, an amphibian, a fish, and an invertebrate. In addition, other embodiments of the animal include a human and a domestic animal. Here, the "domestic animal" means an animal that is raised and bred by a human. Examples of the domestic animal include a non-human mammal (for example, a rodent such as a mouse, a rat, a hamster, or a guinea pig; a lagomorph such as a rabbit; an ungulate such as a pig, a cow, a goat, a horse, or a sheep; a carnivore such as a dog or a cat), a bird (for example, a chicken, a quail, a turkey, a pigeon, a duck, or a goose), a fish (for example, a carp or a goldfish), and an invertebrate (for example, a silkworm or a honeybee).

Herein, the pharmaceutical composition or the food or drink composition may include an additive. Examples of the additive include a physiologically acceptable common blending component such as a carrier, a binder, a stabilizer, an excipient, a diluent, a pH buffering agent, a disintegrant, an isotonic agent, an additive, a coating agent, a solubilizer, a lubricating agent, a gliding agent, a dissolution enhancer, a lubricant, a flavoring agent, a sweetener, a solvent, a gelling agent, or a nutrient. Specific examples of the blending component include water, physiological saline, an animal fat and oil, a vegetable oil, lactose, starch, gelatin, crystalline cellulose, gum, talc, magnesium stearate, hydroxypropyl cellulose, polyalkylene glycol, polyvinyl alcohol, and glycerin.

The S(+) form of MA5 exhibits a physical property different from that of the racemic form, and is particularly easily colored and degraded under a light, acidic, or alkaline conditions. Therefore, the pharmaceutical composition or the food or drink composition of the present invention is preferably stored with protection from light. In addition, the pharmaceutical composition or the food or drink composition of the present invention is preferably stored near neutrality, preferably at pH 6.0 to 8.0. In addition, the S(+) form of MA5 is easily degraded in the presence of a short chain alcohol and chloroform, and thus the pharmaceutical composition or the food or drink composition of the present invention does not include a short chain alcohol and chloroform at the same time (the pharmaceutical composition or the food or drink composition comprises chloroform and no short chain alcohol, comprises the short chain alcohol and no chloroform, or comprises neither a short

chain alcohol nor chloroform), and particularly when the pharmaceutical composition or the food or drink composition includes chloroform, the content of the short chain alcohol based on chloroform is preferably less than 1%.

Herein, the "mitochondrial disease" may be a symptom caused by a decrease in a mitochondrial function such as ATP production, regulation of apoptosis, and regulation of the intracellular concentration of a calcium ion or iron due to a genetic mutation in cell nuclear DNA or mitochondrial DNA or the like, and specific examples thereof include CPEO (chronic progressive external ophthalmoplegia syndrome), MELAS (encephalomyopathy, lactic acidosis, and stroke-like syndrome), MERRF (myoclonic epilepsy with ragged red fibers syndrome), Leigh encephalopathy (subacute necrotizing encephalomyelopathy), Leber disease (Leber hereditary optic neuropathy [LHON]), Kearns-Sayre syndrome (KSS), Barth syndrome, Pearson disease, and Friedreich ataxia (FRDA).

Herein, examples of "the symptom or the disease associated with aging" include thinning hair or hair loss (more specifically, age-related thinning hair or hair loss), hearing loss (more specifically, age-related hearing loss), visual impairment (more specifically, age-related visual impairment), anemia (more specifically, age-related anemia), muscle weakness (more specifically, age-related muscle weakness), and shortened lifespan (more specifically, age-related shortened lifespan).

Herein, examples of the "subject in need of increasing an $NAD^+$ level" or the "subject in need of promoting generation of $NAD^+$" include a subject in need of extending the lifespan (subject in need of longevity); a patient with a neurological disease (Parkinson's disease, depression, Alzheimer's disease, amyotrophic lateral sclerosis [ALS], or the like); a patient with a cardiovascular disease (heart failure, arrhythmia, or the like); a patient with a muscle disease (sarcopenia, inclusion body myositis, muscular dystrophy, or the like); a patient with a renal disease (chronic kidney disease, renal failure, diabetic nephropathy, nephritis, or the like); a patient with a metabolic disease (diabetes, liver dysfunction, alcoholic liver disorder, non-alcoholic fatty liver disease [NAFLD], non-alcoholic steatohepatitis [NASH], thyroid/adrenal gland disease, or the like); a patient with a digestive system disease (inflammatory bowel disease, or the like); a cancer patient; a patient with a symptom or a disease associated with aging (thinning hair or hair loss, hearing loss, visual impairment, anemia, muscle weakness, shortened lifespan, or the like); and a patient with radiation injury, and the pharmaceutical composition or the food or drink composition of the present invention is administered to or ingested by those subjects. The pharmaceutical composition or the food or drink composition of the present invention can increase the $NAD^+$ level (promote generation of $NAD^+$) in cells of those subjects and as a result extend the lifespan or prevent or treat the above diseases and disorders.

Herein, examples of the "subject in need of activating a mitochondrial function" include a patient suffering from a disease related to or caused by a diminished or impaired mitochondrial function, and specific examples thereof include a patient suffering from diabetes, a mitochondrial disease, a brain disease, or the like.

Herein, the administration form of the pharmaceutical composition or the food or drink composition is not particularly limited, and examples thereof include oral administration by administration in a dosage form such as a powder, a granule, a tablet, a capsule, a syrup, or a suspension, and parenteral administration by injection (for example, subcutaneous injection, intravenous injection, or intramuscular injection) in a dosage form such as a solution, an emulsion, or a suspension or by intranasal administration in the form of a spray.

Herein, the dosage (intake) of the S(+) form of MA5 or a physiologically acceptable salt thereof in the pharmaceutical composition or the food or drink composition is appropriately determined depending on the age, the body weight, the sex, the symptom, the sensitivity to an agent, and the like, and is, for example, in the range of 1 μg to 200 mg/kg (body weight)/day. The pharmaceutical composition or the food or drink composition can be administered in a single dose or a plurality of (for example, 2 to 4) divided doses per day.

The present description provides a method for in vitro promoting generation of $NAD^+$ in an animal cell, including culturing an animal cell in the presence of an S(+) form of MA5 or a physiologically acceptable salt thereof. The culture temperature of the animal cell in the above method is usually in the range of 30 to 40°C, and preferably about 37°C (36 to 38°C). In addition, the $CO_2$ concentration during culture is usually in the range of about 1 to 10%, and preferably about 5% (4 to 6%). In addition, the $O_2$ concentration during culture is usually in the range of about 10 to 40%, and preferably about 21% (20 to 22%).

In the above method, examples of the culture medium used when culturing the animal cell include a serum-containing or serum-free culture medium, physiological saline, phosphate-buffered physiological saline, Tris-buffered physiological saline, HEPES-buffered physiological saline, Ringer's solution (lactate Ringer's solution, acetate Ringer's solution, bicarbonate Ringer's solution, or the like), a physiological aqueous solution such as a 5% glucose aqueous solution; and here, examples of the serum-containing culture medium include a culture medium for mammalian cell culture (DMEM, EMEM, IMDM, RPMI-1640, αMEM, F-12, F-10, M-199, AIM-V, or the like) containing 0.1 to 30 (v/v)% serum (fetal bovine serum (FBS), calf bovine serum (CS), or the like), and examples of the serum-free culture medium include the above culture medium for mammalian cell culture supplemented with an appropriate amount (for example, 1 to 30%) of a serum substitute such as commercially available B27 Supplement (-Insulin) (manufactured by Life Technologies Corporation), N2 Supplement (manufactured by Life Technologies Corporation), B27 Supplement (manufactured by Life Technologies Corporation), or Knockout Serum Replacement (manufactured by Invitrogen Corporation).

The present invention provides a method for increasing an $NAD^+$ level in a subject, including administering an S(+) form of MA5 or a physiologically acceptable salt thereof to the subject, wherein the S(+) form of MA5 or the physiologically acceptable salt thereof is substantially free of an R(-) form of MA5 or a physiologically acceptable salt thereof.

In another embodiment, the present invention provides a method for treating or preventing a mitochondrial disease in a subject, including administering an S(+) form of MA5 or a physiologically acceptable salt thereof to the subject, wherein the S(+) form of MA5 or the physiologically acceptable salt thereof is substantially free of an R(-) form of MA5 or a physiologically acceptable salt thereof.

In another embodiment, the present invention provides a method for treating or preventing a symptom or a disease associated with aging, comprising administering an S(+) form of MA5 or a physiologically acceptable salt thereof to a subject, wherein the S(+) form of MA5 or the physiologically acceptable salt thereof is substantially free of an R(-) form of MA5 or a physiologically acceptable salt thereof.

"Substantially free of an R(-) form of MA5 or a physiologically acceptable salt thereof" means that the enantiomeric excess of the S(+) form is about 97% or more, preferably about 97.5% or more, more preferably about 98% or more, particularly preferably about 98.5% or more, and further preferably about 99% or more, for example, about 99.5%, 99.6%, 99.7%, 99.8%, or 99.9% or more.

Hereinafter, the present invention will be described more specifically with reference to Examples, but the technical scope of the present invention is not limited to these Examples. The INS-1E cell line was cultured in an incubator (37°C, 21% $O_2$, 5% $CO_2$) in the presence of RPMI-1640 Medium (manufactured by GIBCO) containing 11.1 mM glucose, 10% FBS, 1 mM sodium pyruvate, 10 mM HEPES, 2 mM glutamine, 50 μM β-mercaptoethanol, penicillin at 100 U/mL, and streptomycin at 100 μg/mL (hereinafter simply referred to as "RPMI-1640 Medium"). In addition, the fibroblast cell line derived from a Leigh encephalopathy patient (MT-ND3 mutant cell line [Leigh synd., m.10191 T>C [p.Ser45Pro]]) was incubated in the above incubator in the presence of DMEM Low Glucose (manufactured by GIBCO) culture medium containing 1% FBS, penicillin at 100 U/mL, and streptomycin at 100 μg/mL (hereinafter referred to as "DMEM Low Glucose culture medium"). In addition, the HBlEpC cell line was incubated in the above incubator in the presence of Human Bladder Epithelial Cell Basal medium (manufactured by Cell Applications, Inc.) culture medium containing 10% FBS, penicillin at 100 U/mL, streptomycin at 100 μg/mL, and HBlEpC Growth Supplement (manufactured by Cell Application's Inc.) (hereinafter referred to as "HBlEpC culture medium").

## Examples

1. Generation of S(+) form of MA5

1-1 Generation of trans-4-(2,4-difluorophenyl)-4-oxo-2-butenoic acid

[Chemical Formula 5]

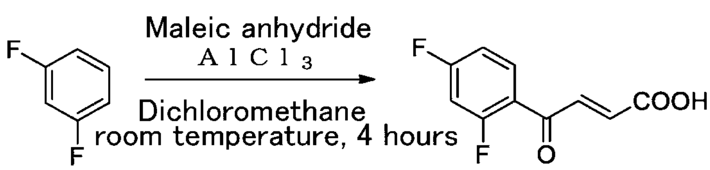


In a 50 mL round bottom flask filled with nitrogen, 1,3-difluorobenzene (0.51 g, 4.47 mmol) was dissolved in dichloromethane (20 mL), maleic anhydride (0.43 g, 4.46 mmol) and aluminum chloride (1.20 g, 9.01 mmol) were added, and the resulting mixture was stirred at room temperature for 4 hours. The reaction solution was adjusted to pH 1 by adding 1 N hydrochloric acid (10 mL), and extracted with ethyl acetate (40 mL) three times. The organic layer was washed with saturated saline and dried over anhydrous sodium sulfate. The solvent was distilled off under reduced pressure, and then the residue was purified by recrystallization with benzene to obtain trans-4-(2,4-difluorophenyl)-4-oxo-2-butenoic acid. (0.57g, yield 56%): melting point 114.8-119.6°C; $^1H$ NMR (acetone-$d_6$): δ 7.98 (m, 1H), 7.71 (dd, $J_{H-F}$ = 15.6, 3.4 Hz, 1H), 7.23 (m, 2H), 6.75 (dd, $J_{H-F}$ = 15.6, 1.2 Hz, 1H); $^{13}C$ NMR (acetone -$d_6$): δ 187.2 (d, $J_{C-F}$= 2.6 Hz), 166.9 (dd, $J_{C-F}$= 254.5, 12.3 Hz), 166.4, 163.4 (dd, $J_{C-F}$ = 254.5, 12.9 Hz), 140.0 (d, $J_{C-F}$ = 6.1 Hz), 134.0 (dd, $J_{C-F}$ = 10.9, 3.6 Hz), 133.0 (d, $J_{C-F}$ = 1.6 Hz), 123.3 (dd, $J_{C-F}$= 12.4, 3.6 Hz), 113.4 (dd, $J_{C-F}$= 21.5, 3.6 Hz), 105.8 (dd, $J_{C-F}$= 27.3, 26.3 Hz); IR (neat): 2917, 1697, 1661 $cm^{-1}$; FAB-MS m/z $[M+H^+]$ calcd for 213 ($C_{11}H_{10}O_3$), found 213.

1-2 Generation of racemic form of MA5

[Chemical Formula 6]

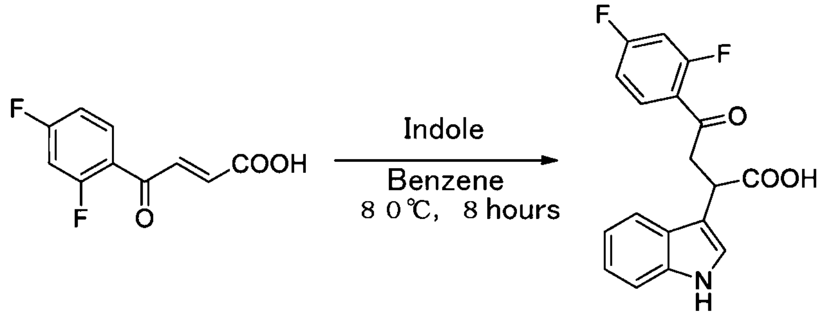


In a 30 mL round bottom flask, trans-4-(2,4-difluorophenyl)-4-oxo-2-butenoic acid (0.39 g, 1.84 mmol) was dissolved in benzene (10 mL), and indole (0.26 g, 2.19 mmol) was added, and the resulting mixture was stirred at 80°C for 8 hours and stirred until the temperature reached room temperature. The reaction solution was distilled off under reduced pressure, and the residue was purified by using silica gel column chromatography (chloroform:methanol = 20:1) to obtain 4-(2,4-difluorophenyl)-2-(1H-indol-3-yl)-4-oxobutanoic acid (racemic form of MA5) (see Table 3). (0.30g, yield 51%): melting point 180.2-184.6°C; $^{1}$H NMR (DMSO-$d_6$): δ 7.98 (m, 1H), 7.65 (d, J = 7.9 Hz, 1H), 7.37 (d, J = 8.1 Hz, 1H), 7.42 (m, 1H), 7.28 (d, J = 2.3 Hz, 1H), 7.24 (m, 1H), 7.09 (t, J = 7.1 Hz, 1H), 7.01 (t, J = 7.5 Hz, 1H), 4.34 (dd, J = 10.5, 3.5 Hz, 1H), 3.90 (ddd, $J_{H\text{-}F}$= 18.5, 10.6, 2.4 Hz, 1H), 3.30 (ddd, $J_{H\text{-}F}$= 18.5, 6.1, 3.5 Hz, 1H); $^{13}$C NMR (DMSO-$d_6$): δ 195.2 (d, $J_{C\text{-}F}$ = 4.1 Hz), 174.8, 165.2 (d, $J_{C\text{-}F}$ = 253.0, 13.4 Hz), 162.2 (d, $J_{C\text{-}F}$ = 255.5, 13.4 Hz), 136.4, 132.7 (dd, $J_{C\text{-}F}$ = 10.8, 4.1 Hz), 126.3, 123.3, 122.2 (dd, $J_{C\text{-}F}$ = 12.3, 3.6 Hz), 121.4, 119.1, 118.8, 112.6 (dd, $J_{C\text{-}F}$ = 21.1, 3.6 Hz), 111.9, 111.8, 105.4 (dd, $J_{C\text{-}F}$ = 26.1 Hz), 45.6 (d, $J_{C\text{-}F}$ = 6.3 Hz), 37.9; IR (neat): 3382, 2919, 1678 $cm^{-1}$; HRFAB m/z $[M+H]^+$calcd for 332.1036 ($C_{19}H_{17}NO_3$), found 312.1028.

1-3 Preparative separation of S(+) form of MA5

Preparative separation of the S(+) form of MA5 from the racemic form of MA5 synthesized was carried out by using preparative separation apparatus LC-Forte (manufactured by YMC Co., Ltd.). In addition, as a preparative column, CHIRALPAK AD-H (column inner diameter of 2 cm, column length of 25 cm, manufactured by Daicel Corporation) was used. The S(+) form was eluted over 40 minutes at a flow rate of 10 mL/min by using solution A (0.05% TFA/hexane), solution B (0.05% TFA/2-propanol), and a mobile phase having a single composition of solution A/solution B = 60/40. The flow rate of the mobile phase can also be 20 mL/min, and the elution time can also be 20 minutes, which is half of the above time. The detection wavelength for MA5 was set to 220 nm. The retention times of the S(+) form and the R(-) form of MA5 (see Table 3) were 17.3 minutes and 29.8 minutes (flow rate of 10 mL/min), respectively. The S(+) form of MA5 was obtained with an optical purity of 100% by column separation. Chromatograms are shown in Figure 17.

1-4 Determination of absolute configuration of S(+) form of MA5

Measurement of the optical rotations of MA5 in the pre-elution fraction and MA5 in the post-elution fraction preparatively separated was carried out by using polarimeter P2200 (manufactured by JASCO Corporation).

The specific rotation of MA5 in the pre-elution fraction was $[\alpha]_D$ + 130.3 (c = 1.0, chloroform solution, 25°C), and the specific rotation of MA5 in the post-elution fraction was $[\alpha]_D$ - 128.6 (c = 1.0, chloroform solution, 25°C).

X-ray crystallography was carried out by using AXS "SMART APEX II" (manufactured by Bruker Corporation). For the crystals used for measurement, crystals of benzylamine salts of MA5 in the respective elution fractions were prepared because they are suitable for single crystal formation (see Tables 4 and 5). Crude crystals were obtained from an ethyl acetate solution prepared by mixing MA5 in the pre-elution fraction or MA5 in the post-elution fraction and benzylamine were mixed at a molar ratio of 1:1, and then benzylamine salts of MA5 in the respective elution fractions were obtained by recrystallization from a water-methanol solution. The crystal structures were determined by the anomalous scattering effect, and the absolute configuration of the benzylamine salt of MA5 in the pre-elution fraction was S, whereas the absolute configuration of the benzylamine salt of MA5 in the post-elution fraction was R.

[Table 1]

| Crystal data on S(+)-MA5 benzylamine salt: |
|---|
| $C_{25}H_{22}F_2N_2O_3$ (*M* =436. 44 g/mol), monoclinic, space group $P2_1$ (no. 4), *a* = 11.0175 (4) Å, *b* = 6. 6046 (2) Å, *c* = 15. 0931 (5) Å, $\beta$ = 106. 9980(10)° , *V* = 1050. 29 (6) $Å^3$, *Z* = 2, *T* = 293. 15 K, $\mu$(CuK$\alpha$) = 0. 860 $mm^{-1}$, *Dcalc* = 1.380 g/$cm^3$, crystal size 0.2 × 0.15 × 0.15 $mm^3$, F(000) = 456, 35841 reflections measured (8.826° ≤ 2Θ ≤ 149.292° ), 4210 unique ($R_{int}$ = 0. 0369, $R_{sigma}$ = 0. 0255) which were used in all calculations. The final $R_1$ was 0.0263 (I > 2 σ (I)) and $wR_2$ was 0.0699 (all data). The goodness of fit on $F^2$ was 1.082. Flack parameter = 0.00(2). |

[Table 2]

| Crystal data on R(-)-MA5 benzylamine salt: |
|---|
| $C_{25}H_{22}F_2N_2O_3$ (*M* =436. 44 g/mol), monoclinic, space group $P2_1$ (no. 4), *a* = 11.0185(3) Å, *b* = 6. 6030(2) Å, c = 15. 0857(5) Å, $\beta$ = 106. 9090(10)° , *V* = 1050. 11(6) $Å^3$, *Z = 2, T* = 100(2) K, $\mu$ (CuK$\alpha$) = 0.860 $mm^{-1}$, *Dcalc* = 1.380 g/$cm^3$, crystal size 0.2 × 0.1 × 0.1 $mm^3$, F(000) = 456, 21786 reflections measured (8.832° ≤ 2Θ ≤ 149.224° ), 4116 unique ($R_{int}$ = 0.0308, $R_{sigma}$ = 0.0254) which were used in all calculations. The final $R_1$ was 0.0263 (1 > 2 σ (1)) and $wR_2$ was 0.0677 (all data). The goodness of fit on $F^2$ was 1.066. Flack parameter = 0.036(18). |

From the above results, it was determined that MA5 in the pre-elution fraction was the S(+) form and that MA5 in the post-elution fraction was the R(-) form.

[Table 3]

| Compound name | Structural formula |
|---|---|
| Racemic form of MA5 | 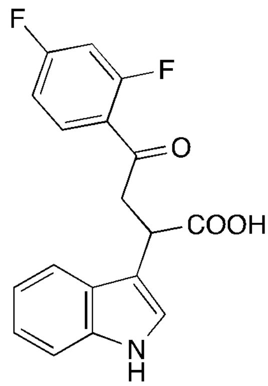 |
| S(+) form of MA5 | 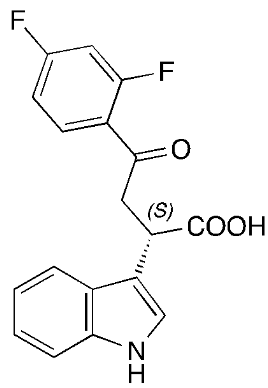 |

(continued)

| Compound name | Structural formula |
|---|---|
| R(-) form of MA5 | 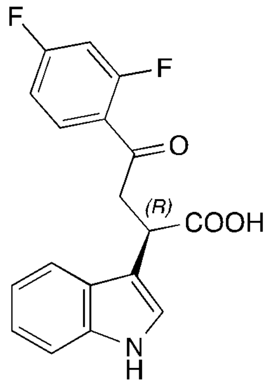 |

[Table 4]

| Compound name | Structural formula |
|---|---|
| Benzylamine salt of S(+) form of MA5 | 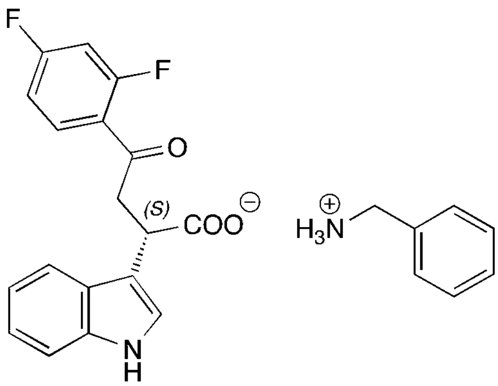 |
| Benzylamine salt of R(-) form of MA5 | 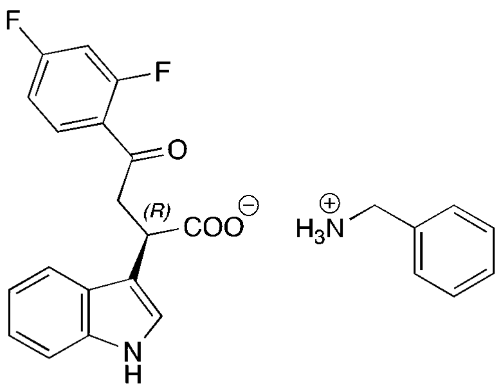 |

[Table 5]

| Compound name | Structural formula |
|---|---|
| ORTEP diagram of benzylamine salt of S(+) form of MA5 | 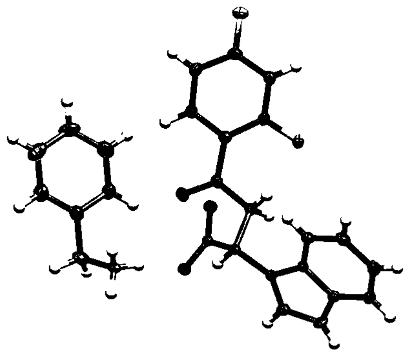 |
| ORTEP diagram of benzylamine salt of R(-) form of MA5 | 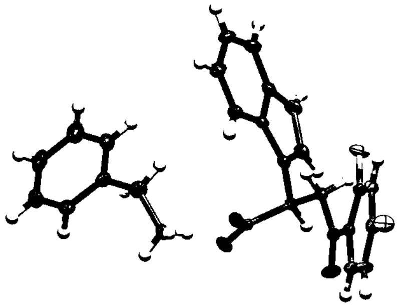 |

2. Evaluation of amount of $NAD^+$ in animal cells

In order to investigate the influence of the S(+) form of MA5 on the amount of $NAD^+$ in animal cells, analysis was carried out according to the method described in the section "2-1" below.

2-1 Method

An INS-1E cell line (see the literature "Drug Metab. Pharmacokinet. 25 (3): 274-282 (2010)") was seeded in a lysine-coated 96-well plate at $2 \times 10^4$ cells per well, and cultured for 48 hours in the presence of RPMI-1640 Medium, then the Medium was removed, RPMI-1640 Medium containing 0.1% DMSO, which is a solvent for the compounds, or RPMI-1640 Medium containing various concentrations (3 pM, 10 pM, 30 pM, 100 pM, 300 pM, 1 nM, or 3 nM) of three compounds (racemic form of MA5, S(+) form of MA5, or R(-) form of MA5) were added in an amount of 100 $\mu$L each, and the cells were cultured for 6 hours. After that, the amount of $NAD^+$ in the cells was measured by using NAD/NADH-Glo Assay (G9071, manufactured by Promega Corporation).

2-2 Results

Even when the INS-1E cell line was cultured in the presence of the racemic form of MA5, the amount of $NAD^+$ in the cells remained almost unchanged as compared with when the INS-1E cell line was cultured in the absence of the racemic form of MA5 (see Figure 1A). On the other hand, when the INS-1E cell line was cultured in the presence of the R(-) form of MA5, the amount of $NAD^+$ in the cells decreased in a culture time-dependent manner as compared with when the INS-1E cell line was cultured in the absence of the R(-) form of MA5 (see Figure 1C). In addition, when the INS-1E cell line was cultured in the presence of the S(+) form of MA5, the amount of $NAD^+$ in the cells increased in a culture time-dependent manner as compared with when the INS-1E cell line was cultured in the absence of the S(+) form of MA5 (see Figure 1B).

These results indicate that the R(-) form of MA5 has the action of decreasing the amount of $NAD^+$ in animal cells, whereas on the contrary, the S(+) form of MA5 has the action of increasing the amount of $NAD^+$ in animal cells (that is, the action of promoting generation of $NAD^+$ in animal cells).

3. In vivo administration test 1

When the S(+) form of MA5 was administered in vivo, whether or not chiral conversion occurred and changes in

the concentration of the S(+) form of MA5 in plasma were analyzed according to the method described in the section "3-1" below.

3-1 Method

Each enantiomer (S(+) form or R(-) form) of MA5 was dissolved in a mixed solution of physiological saline and 0.1 N sodium hydroxide (9:1), and filtered and sterilized by using a 0.22 μm filter to prepare a solution containing 2 mL/kg of each enantiomer of MA5. Next, the solution containing each enantiomer of MA5 was intravenously administered to each of three 4- to 5-year-old male cynomolgus monkeys at a single dose of 1 mg/kg. As a control, a solution containing no enantiomer of MA5 was also administered in the same manner. Blood was collected 5 minutes, 15 minutes, 30 minutes, 1 hour, 2 hours, 4 hours, 6 hours, and 24 hours after administration to collect plasma, the concentration of each enantiomer of MA5 in the plasma was measured by LC-MS/MS (liquid chromatography/tandem mass spectrometry), and the area under the blood concentration-time curve (AUC) (μg·hr/mL) was calculated. The analysis conditions for LC-MS/MS are as follows.

As an ionization unit, an apparatus in which NANOSPACESI-2 (manufactured by OSAKA SODA CO., LTD.) as a high-performance liquid chromatograph was connected to a triple quadrupole mass spectrometer (TSQ Quantum Ultra, manufactured by Thermo Fisher Scientific Inc.) equipped with an ESI probe was used. The S(+) form of MA5 (S-(+)-MA5) and the R(-) form of MA5 (R-(-)-MA5), which are substances to be analyzed, were subjected to quantitative analysis by the internal standard method using their respective stable isotopes S-(+)-MA5-$d_6$ and R-(-)-MA5-$d_6$. CHIRALPAK AD-3R (150 × 2.1 mm i.d., 3.0 μm particle size, manufactured by Daicel Corporation) was used as the analytical column, and CAPCELL C8UG120 (10 × 2.0 mm i.d., 5.0 μm particle size, manufactured by OSAKA SODA CO., LTD.) was used as the guard column. The column oven temperature was 35°C. Formic acid/water (0.1/100 [v/v]) (solution A)/acetonitrile (solution B) was used as the mobile phase, and the flow rate was 200 μL/min. The measurement was carried out under an isocratic condition in which a single composition of solution A/solution B (57/43 [v/v]) was used for elution. Solution A/solution B (57/43 [v/v]) was used until the analysis time reached 9.5 minutes, and solution A/solution B (10/90 [v/v]) was used after the analysis time reached 9.7 minutes. In addition, a system for washing the column with solution A/solution B (10/90 [v/v]) was incorporated while the analysis time elapsed was 9.7 minutes to 11.7 minutes, and the column was equilibrated with solution A/solution B (57/43 [v/v]) while the analysis time elapsed was 11.8 minutes to 15 minutes. Retention times were 6.95 minutes for S-(+)-MA5, 6.80 minutes for S-(+)-MA5-$d_6$, 8.69 minutes for R-(-)-MA5, and 8.56 minutes for R-(-)-MA5-$d_6$.

The MS conditions were detected in negative ion mode, a spray voltage set to 2.5 kV, a vaporizer temperature set to 250°C, and an ion transfer tube temperature set to 250°C. The precursor ion>product ion SRM channel was set to "m/z 328.0>116.2" for MA5 and "m/z 334.0>121.1" for MA5-$d_6$. The collision energy for generating a product ion was set to 12 eV for MA5 and 17 eV for MA5-$d_6$. Waveform analysis and quantitative calculation by the internal standard method were carried out by using Xcalibur (manufactured by Thermo Fisher Scientific Inc.).

3-2 Results

From immediately after administration of the S(+) form of MA5 (5 minutes after administration) to 24 hours after administration when the S(+) form of MA5 disappeared in blood, the R(-) form of MA5 was not detected, and only the S(+) form of MA5 was detected. This result indicates that the S(+) form of MA5 does not cause chiral conversion in vivo.

In addition, the AUC value when the R(-) form of MA5 was administered was 12 (μg·hr/mL), whereas the AUC value when the S(+) form of MA5 was administered was 30 (μg·hr/mL) (see Table 6). This result indicates that the S(+) form of MA5 is more than 2.5 times more likely to be retained in blood than the R(-) form of MA5, suggesting that the dosage of the S(+) form of MA5 can be made lower than that of the R(-) form of MA5.

[Table 6]

| | $t_{1/2}$ (hr) | ke ($hr^{-1}$) | AUC (μg/mL•hr) |
|---|---|---|---|
| S(+) form of MA5 | 2.4 | 0.29 | 30 |
| R(-) form of MA5 | 1.1 | 0.63 | 12 |

4. Metabolism test using human liver microsomes

The S(+) form of MA5 was more likely to be retained in blood than the R(-) form of MA5 in vivo, and thus whether or not there was a difference in metabolic rate between the enantiomers of MA5 was analyzed according to the method described in the section "4-1" below.

4-1 Method

The S(+) form or the R(-) form of MA5 was dissolved in acetonitrile (for LC-MS, manufactured by Kanto Chemical Co., Inc.) and each adjusted to 10 mM. 855 mg of sucrose (manufactured by FUJIFILM Wako Pure Chemical Corporation) was dissolved in 10 mL of water to prepare a 250 mM sucrose solution, and then by using this solution, a liquid containing 20 mg/mL human liver microsomes (8-donor-pool, manufactured by Sekisui XenoTech, LLC) was diluted 4-fold to prepare a 5 mg/mL human liver microsome suspension. In a 1 mL tube, 5 μL of three test solutions (sucrose solution containing 10 mM S(+) form of MA5, sucrose solution containing 10 mM R(-) form of MA5, or control Vehicle [sucrose solution]), 5 μL of NADPH Regenerating System Solution B (manufactured by Corning Incorporated), 100 μL of UGT Reaction Mix Solution B (manufactured by Corning Incorporated), and 275 μL of water were added to 50 μL of the human liver microsome suspension, and the resulting mixture was shaken for 30 seconds and preincubated at 37°C for 5 minutes. After that, 25 μL of NADPH Regenerating System Solution A (manufactured by Corning Incorporated) and 40 μL of UGT Reaction Mix Solution A (manufactured by Corning Incorporated) were added, and the resulting mixture was shaken for 30 seconds and incubated at 37°C for 1 hour. The reaction was stopped by adding 500 μL of an ice-cold acetonitrile solution containing 0.1% formic acid, and the resulting mixture was shaken for 30 seconds, sonicated for 10 minutes, and then centrifuged at 20,000 × g at 4°C for 10 minutes. 50 μL of the supernatant was collected in a fresh 1 mL tube, centrifugally concentrated at 2,000 rpm at room temperature (25 to 35°C) for 30 minutes, and frozen at -80°C. This was dried with a freeze dryer for 30 minutes and redissolved in 25 μL of 20% methanol containing 0.1% formic acid (for LC-MS, manufactured by Kanto Chemical Co., Inc.), and then 2 μL thereof was subjected to LC/HRMS. The number of samples was n = 3, and operations were carried out on ice as much as possible.

LC/HRMS measurement

Nanospace si-2 (manufactured by OSAKA SODA CO., LTD.) was used as an LC apparatus. Develosil C30-ug-3 (2.1 × 150 mm, 3.0 μm particle size, manufactured by Nomura Chemical Co., Ltd.) was used as a column, 10 mM $NH_5CO_3/H_2O$ was used as the mobile phase solution A, methanol was used as the mobile phase solution B, and linear gradient elution was carried out at a flow rate of 200 μL/min (15 to 65% solution B (0.0 to 22.0 minutes), 65 to 90% solution B (22.0 to 24.4 minutes), 100% solution B (24.5 to 29.0 minutes), 15% solution B (30.0 to 40.0 minutes)). The column oven was set to 40°C, the sample injection volume was set to 2 μL, 10% methanol was selected as the wash solution, and 50% acetonitrile was selected as the wash port solution.

Q-Exactive (manufactured by Thermo Fisher Scientific Inc.) was used as an HRMS apparatus. The ESI conditions set were spray voltage: 3000 V, sheath gas pressure: 40 psi, auxiliary gas pressure: 10 psi, and capillary temperature/vaporizer temperature: 300°C. Full scan-ddMS$^2$ (topN) mode for both positive and negative ions was selected for detection. Various parameters are as follows: Full scan; Resolution 70,000, AGC target 3e6, Maximum IT 100 ms, Scan range 80 to 1,200, ddMS$^2$; Resolution 17,500, AGC target 5e4, Maximum IT 50 ms, topN N=20, Isolation window 4.0 m/z, and NCE 20 (50% stepped NCE). Additional MS$^2$ data were measured in target MS$^2$ mode for both positive and negative ions. Various parameters are as follows: Resolution 35,000, AGC target 6e5, Maximum IT 200 ms, Isolation window 4.0 m/z, and NCE 20 (50% stepped NCE).

Data analysis

Data analysis was carried out by using Compound Discoverer (manufactured by Thermo Fisher Scientific Inc.) and Xcalibur (manufactured by Thermo Fisher Scientific Inc.). Metabolism w/ FISh Workflow was selected for peak extraction, and structural information of MA5 was registered in "Compound." Min. Peak Intensity was set to 1/10,000 of the MA5 average peak intensity in each sample. $[M+Na]^+$, $[M+NH_4]^+$, $[M-H_2O+H]^+$, and $[M+Cl]^-$ were added to Ion Adducts of Predict Metabolites Node. In addition, tryptophan>kynurenine pathway [-C+O], AMP conjugation [$+C_{10}H_{12}N_5O_6P$], CoA conjugation [$+C_{21}H_{34}N_7O_{15}P_3S$] were added to Metabolic Pathways.

4-2 Results

When human liver microsomes were incubated in the presence of the racemic form of MA5 or the R(-) form of MA5, glucuronide conjugates (+C6H8O6 [+1.7 min, +2.6 min]) were detected, whereas when human liver microsomes were incubated in the presence of the S(+) form of MA5, such glucuronide conjugates were detected only at a level that was little or no different from the control (see Figure 2).

This result indicates that the R(-) form of MA5 is no less than 10 times more likely to be glucuronidated than the S(+) form of MA5, supporting the results of the section "4. Metabolism test using human liver microsomes" above, that is, the result that the S(+) form of MA5 was more likely to be retained in blood than the R(-) form of MA5 in vivo.

Furthermore, as a result of carrying out a 4-week repeated dose toxicity test in rats and a 4-week repeated dose

toxicity test in monkeys from GLP-compliant non-clinical studies, a finding of toxicity to the urothelium was observed in the high-dose administration group. In order to clarify the cause thereof, urine metabolites were evaluated, and the urine concentrations of glucuronide conjugates of the racemic form of MA5 in monkeys and the urine concentrations of sulfate conjugates of the racemic form of MA5 in rats correlated with the development of toxicity, and it was presumed that these conjugates were responsible for toxicity.

It has been revealed that there are species differences between monkeys and rats in liver metabolic enzymes and renal transporters, and that hepatic metabolism in humans is similar to that in monkeys, and thus it is presumed that a glucuronide conjugate is also responsible for a side effect in humans. From the metabolism test using human liver microsomes, the S(+) form of MA5 produces almost no (1/10 or less) glucuronide conjugate as compared with the R(-) form, and thus it can be deemed that the S(+) form of MA5 is a safer compound with less side effects than the R(-) form of MA5.

5. Cytotoxicity evaluation 1

In order to evaluate the toxicity of the S(+) form of MA5 to animal cells, analysis was carried out according to the method described in the section "5-1" below.

5-1 Method

An HBlEpC cell line (manufactured by Cell Application) was seeded in a 96-well plate at $2 \times 10^3$ cells per well, 0.1% DMSO, which is a solvent for the compounds, or various concentrations (30 nM, 100 nM, 300 nM, 1 μM, 3 μM, 10 μM, 30 μM, 100 μM, 300 μM, or 1 mM) of three compounds (racemic form of MA5, S(+) form of MA5, or R(-) form of MA5) were added to the HBlEpC culture medium, and the cells were cultured for 48 hours. As a control, an experiment in which nothing was added was also carried out ("-" in Figure 3). The viable cell level (viable cell count level) after culture was measured by a WST-8 assay using Cell Count Reagent SF (manufactured by Nacalai Tesque, Inc.).

5-2 Results

When the HBlEpC cell line was cultured in the presence of the racemic form or an enantiomer (S(+) form or R(-) form) of MA5, the viability of the cells was higher than when the HBlEpC cell line was cultured in the presence of the racemic form of MA5, and in particular, the viability of the cells was higher with the S(+) form of MA5 than with the R(-) form of MA5 (see Figure 3). This result indicates that the S(+) form of MA5 has lower cytotoxicity to animal cells than the racemic form of MA5 and the R(-) form of MA5.

6. Cytotoxicity evaluation 2

In order to evaluate the toxicity of the S(+) form of MA5 to animal cells, analysis was carried out according to the method described in the section "6-1" below.

6-1 Method

An MT-ND3 mutant cell line was seeded in a 96-well plate at $2 \times 10^3$ cells per well and cultured for 2 days in the presence of DMEM Low Glucose culture medium, then 0.1% DMSO, which is a solvent for the compounds, or various concentrations (0.003 μM, 0.01 μM, 0.03 μM, 0.1 μM, 0.3 μM, 1 μM, 3 μM, 10 μM, 30 μM, or 100 μM) of each enantiomer (S(+) form or R(-) form) of MA5 was added, the cells were further cultured for 5 days, and then the cell survival level was measured. Specifically, the cell survival level was measured by an MTT assay using Cell Counting Kit-8 (manufactured by Dojindo Laboratories). That is, 100 μL of Cell Count Reagent SF was added to each well, incubated for 2 hours, and stirred for 3 seconds with a microplate reader, and the absorbance at 450 nm (reference: 750 nm) was measured (see Figure 4). As a control, an experiment in which nothing was added was also carried out ("-" in Figure 4).

6-2 Results

Even when the MT-ND3 mutant cell line was cultured in the presence of the S(+) form of MA5 at high concentrations (30 μM and 100 μM), no decrease in viable cell level was observed (see Figure 4A). On the other hand, when the MT-ND3 mutant cell line was cultured in the presence of the R(-) form of MA5 at high concentrations (30 μM and 100 μM), a significant decrease in viable cell level was observed (see Figure 4B). These results indicate that the S(+) form of MA5 has lower cytotoxicity to animal cells than the R(-) form of MA5, supporting the results of the section "5. Cytotoxicity evaluation 1" above.

7. Evaluation of ability to produce ATP in animal cells

It has been reported that the racemic form of MA5 has the action of enhancing the ability to produce ATP in animal cells (see, for example, International Publication No. WO 2014/080640). Therefore, in order to check whether or not there is a difference in the action of enhancing the ability to produce ATP between the S(+) form of MA5 and the R(-) form of MA5, analysis was carried out according to the method described in section "7-1" below.

7-1 Method

An MT-ND3 mutant cell line was seeded in a 96-well plate at $2 \times 10^3$ cells per well and cultured for 24 hours in the presence of DMEM Low Glucose culture medium, then 0.1% DMSO, which is a solvent for the compounds, or various concentrations (0.1 μM or 1 μM) of three compounds (racemic form of MA5, S(+) form of MA5, or R(-) form of MA5) were added, and the cells were cultured for 6 hours, and then the concentration of ATP produced in the culture medium was measured with GloMa 96 Microplate Luminometer (Promega Corporation) by using a "cellular" ATP measurement reagent (manufactured by TOYO B-Net Co., Ltd.).

7-2 Results

No difference was observed in the level of ATP production in animal cells between the S(+) form of MA5 and the R(-) form of MA5 (see Figure 5).

8. Evaluation of lifespan

In order to confirm that the S(+) form of MA5 has the effect of extending the lifespan of an organism, analysis is carried out, for example, according to the methods described in the sections [Method 1] and [Method 2] below.

[Method 1]

[1] Neurodegeneration model flies (obtained from the Bloomington Drosophila Stock Center) are crossed, and the hatched eggs are raised at 18°C.
[2] One to three days after emergence into adults, the adults are placed in an incubator at 29°C.
[3] Vials containing a 5% sucrose solution or a 5% sucrose solution containing various concentrations (100 nM or 1000 nM) of three compounds (racemic form of MAS, S(+) form of MAS, or R(-) form of MAS) are provided, and 20 male (♂) flies and female (♀) flies are transferred to each vial and allowed to ingest each solution (for 20 hours at 29°C, with protection from light).
[4] The flies are transferred to another vial containing regular food and allowed to ingest the regular food (for 1 day).
[5] From the next day onward, the above steps [3] and [4] are repeated.
[6] In the above steps [3] and [4], the dead flies are removed, the number thereof is recorded, and the lifespan of the dead flies is recorded. Once the vial is emptied (that is, there are no live flies in the vial), the number of survivors is calculated by taking the sum of the recorded numbers of dead flies as the original number, and the average lifespan of the flies is calculated based on the lifespans of the dead individual flies and the total number of surviving flies. The reason for changing the temperature from 18°C to 29°C is that a temperature-sensitive gene expression system is used, and that no neurodegenerative proteins are expressed during the developmental process, and neurodegenerative proteins are expressed after emergence into adults (expression of neurodegenerative proteins in all neurons during the developmental process is often lethal).

Flies have been used as a model organism for a human disease (see the literature "Hum Mol Genet. 2019. PMID: 31227826") or used as a model organism for human aging (see the literature "FEBS Open Bio. 2022. PMID: 34854258"). Because of this, if the lifespan extension effect of the S(+) form of MAS is observed in flies, it can be deemed that the effect can also be applied to a human.

[Method 2]

[1] 28-day-old α-klotho homo KO mice (male [♂], 4 weeks old, n = 3, 2 repeats) are obtained from CLEA Japan, Inc.

and acclimatized to the environment for 2 days.

[2] On day 30 after birth, three compounds (racemic form of MAS, S(+) form of MAS, or R(-) form of MAS) suspended in water are orally administered to the above KO mice at a dose of 10 mg/kg body weight/100 μL/day.

[3] The body weights of the KO mice are measured once a week and recorded until the death thereof is confirmed. As a control, the body weights of KO mice to which the above three compounds have not been orally administered are similarly measured until the death thereof is confirmed.

9. Evaluation of mitochondrial function

It has been reported that in immt-1-deficient mutant C. elegans (hereinafter referred to as "immt-1/mitofilin mutant"), which is a C. elegans ortholog of the mitochondrial inner membrane protein (Mitofilin), mitochondria in muscle cells swell (see the literature "Journal of Cellular Physiology. 224 (2010) 748-756" and the literature "Molecular Biology of the Cell. 22 (2011) 831-841."). Therefore, when three compounds (racemic form of MAS, S(+) form of MAS, or R(-) form of MAS) were administered to immt-1/mitofilin mutants, whether or not mitochondrial swelling was alleviated was analyzed.

Specifically, immt-1/mitofilin mutants (NBRP [National Bio Resource Project] C. elegans [Tokyo Women's Medical University]) of ccIs4251 (Pmyo-3::GFP::LacZ::NLS, Pmyo-3::mitochondrial-GFP+dpy-20(+), the literature "Cell 139 (2009) 623-633") genetically engineered to be able to visualize mitochondrial DNA and genomic DNA with GFP were raised for 5 days from the L4 larval stage (that is, until these became 4-day-old adults) on a culture plate (E. coli OP-50 NGM agar medium) that was sprayed with the control DMSO (solvent), or the above culture plate that was sprayed with three compounds (racemic form of MAS, S(+) form of MAS, or R(-) form of MAS) such that the final concentration was 10 μM, body wall muscle cells were observed in the 4-day-old adults (see Figure 6), and the number of abnormal mitochondria (swelling mitochondria) and the proportion of body wall muscle cells in which abnormal mitochondria were observed were measured (see Figures 7 and 8). As a comparative control, ccIs4251 wild-type C. elegans (obtained from CGC [Caenorhabditis Genetics Center]) was raised on a culture plate and analyzed in the same manner.

As a result, when the immt-1/mitofilin mutants were raised in the presence of the S(+) form of MAS, the proportion of body wall muscle cells in which 5 or more swelling mitochondria per body wall muscle cell were observed greatly decreased (see Figure 7), and the number of swelling mitochondria per body wall muscle cell also greatly decreased (see Figure 8), as compared with when the mutants were raised in the presence of the control DMSO and when the mutants were raised in the presence of the R(-) form of MA5. In addition, when the immt-1/mitofilin mutants were raised in the presence of the R(-) form of MA5, the proportion of body wall muscle cells in which 5 or more swelling mitochondria per body wall muscle cell were observed and the number of swelling mitochondria per body wall muscle cell remained almost unchanged or only slightly decreased, as compared with when the mutants were raised in the presence of the control DMSO (see Figures 7 and 8).

These results indicate that the R(-) form of MA5 has almost no action of alleviating mitochondrial swelling, whereas the S(+) form of MA5 has the action of alleviating mitochondrial swelling. That is, the results indicate that the S(+) form of MA5 has the action of improving a mitochondrial function in C. elegans. In addition, C. elegans has been used as a model organism for analyzing a human mitochondrial function (see the literature "Nature. 2018. PMID: 30356218"), and thus it can be deemed that the S(+) form of MA5 has been shown to be useful as a mitochondrial function activating agent and a therapeutic agent or a preventive agent for a mitochondrial disease in a human.

10. In vivo administration test 2

In "3. In vivo administration test 1" above, it was confirmed that the intravenously administered S(+) form of MA5 is more likely to be retained in blood than the intravenously administered R(-) form of MA5, and whether or not the same results were obtained even when these were orally administered was checked. Specifically, according to the method described in "3-1 Method" above, a solution containing each enantiomer (S(+) form or R(-) form) of MA5 was orally administered to male cynomolgus monkeys at a single dose of 100 mg/kg, the concentration of each enantiomer of MA5 and the concentration of a reduced metabolite of each enantiomer of MA5 (the compound represented by formula (B) [that is, Red-MA5]) in plasma were measured.

[Chemical Formula 7]

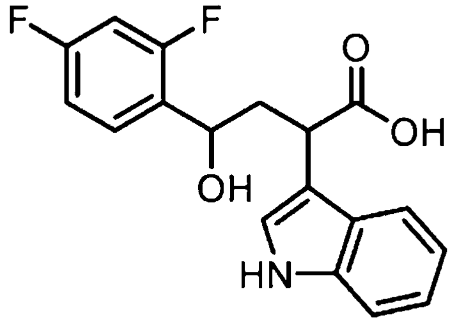

(B)

As a result, after oral administration, the concentration of the S(+) form of MA5 in plasma was higher than that of the R(-) form of MA5 in plasma (see Figure 9). In addition, the reduced metabolite (the compound represented by formula (B), that is, Red-MA5) in plasma was detected when the R(-) form of MA5 was orally administered, whereas almost no reduced metabolite was detected when the S(+) form of MA5 was orally administered (see Figure 10).

These results indicate that even when the S(+) form of MA5 is orally administered, the S(+) form is more likely to be retained in blood than is the R(-) form when the R(-) form of MA5 is orally administered, and the S(+) form of MA5 is less likely to be metabolized than the R(-) form of MA5, and thus the S(+) form of MA5 can exert the effect thereof in vivo more sustainably than the R(-) form of MAS.

11. Cytotoxicity evaluation 3

In order to evaluate the toxicity of Red-MAS to animal cells, analysis was carried out according to the method described in the section "11-1" below.

11-1 Method

A KCMC10 cell line was seeded in a 96-well plate at $3 \times 10^3$ cells per well, and cultured for 24 hours in the presence of 0.1% DMSO, which is a solvent for the compounds, or various concentrations (3 nM, 10 nM, 30 nM, 100 nM, 300 nM, 1 μM, 3 μM, 10 μM, or 30 μM) of two compounds (racemic form of MAS or Red-MAS), and then the cell survival level was measured. Specifically, the cell survival level was measured by an MTT assay using Cell Counting Kit-8 (manufactured by Dojindo Laboratories). That is, 100 μL of Cell Count Reagent SF was added to each well, incubated for 2 hours, and stirred for 3 seconds with a microplate reader, and the absorbance at 450 nm (reference: 750 nm) was measured (see Figure 11). As a control, an experiment in which nothing was added was also carried out ("-" in Figure 11).

11-2 Results

Even when the KCMC10 cell line was cultured in the presence of the 30 μM racemic form of MAS, no decrease in viable cell level was observed (see Figure 11A), whereas when the KCMC10 cell line was cultured in the presence of 30 μM Red-MAS, a significant decrease in viable cell level was observed (see Figure 11B). This result indicates that MAS has lower cytotoxicity than Red-MAS. That is, the result, combined with the results of "10. In vivo administration test 2" above, indicates that the S(+) form of MAS administered in vivo has lower cytotoxicity than the R(-) form of MAS administered in vivo.

12. Evaluation of SIRT expression level in animal cells

It is known that there is a positive correlation between the amount of $NAD^+$ and the SIRT expression level in cells (see the literature "Nat Rev Nephrol. 2017. PMID: 28163307"). In addition, it has been reported that the increased expression of an SIRT in inner ear cells is associated with improvement in hearing loss (see the literature "Front Cell Dev Biol. 2021. PMID: 34869361," the literature "Exp Cell Res. 2022 Jul 11:113280.", and the literature "Hindawi Neural Plasticity Volume 2021, Article ID 5520794"). Therefore, in order to investigate whether or not when the S(+) form of MAS increased the amount of $NAD^+$ in cells, the expression level of an SIRT in inner ear cells was increased, and this was useful for improving hearing loss, analysis was carried out according to the method described in the section "12-1" below.

12-1 Method

A mouse inner ear cell line (HEI-OC1 cell line) was seeded in a dish containing 10 mL of a culture medium (low glucose DMEM medium [glucose concentration of 1.0 g/dL] supplemented with 10% by weight of FBS) at $1 \times 10^6$ cells/dish and cultured. One day after, 0.1% DMSO, which is a solvent for the compounds, or various concentrations (1 μM, 3 μM, 10 μM, or 30 μM) of three compounds (racemic form of MAS, S(+) form of MAS, or R(-) form of MAS) were added, and the cells were cultured for 24 hours. After the culture was completed, the cells were collected, the cells were disrupted by a conventional method, and the expression levels of various SIRTs (SIRT1, SIRT2, SIRT3, SIRT5, SIRT6, and SIRT7) in the cells were analyzed by Western blotting (see Figures 12 to 14 [Figure 12-1 to Figure 14-2]).

12-2 Results

Even when the HEI-OC1 cell line was cultured in the presence of the R(-) form of MAS, the expression levels of the various SIRTs (SIRT1, SIRT2, SIRT3, SIRT5, SIRT6, and SIRT7) in the cells remained almost unchanged as compared with when the HEI-OC1 cell line was cultured in the absence of the R(-) form of MAS (see Figure 14 [Figure 14-1 and Figure 14-2]), whereas when the HEI-OC1 cell line was cultured in the presence of the S(+) form of MAS, the expression levels of the various SIRTs in the cells increased as compared with when the HEI-OC1 cell line was cultured in the absence of the S(+) form of MAS (see Figure 13 [Figure 13-1 and Figure 13-2]).

These results, combined with the results of "2. Evaluation of amount of $NAD^+$ in animal cells" above, indicate that the S(+) form of MAS increased the amount of $NAD^+$ in the cells, and as a result, the expression levels of the SIRTs in the cells increased, and this is useful for improving hearing loss.

13. Evaluation of motor function in animal

In order to confirm that the S(+) form of MAS has the effect of improving motor dysfunction in an animal, for example, the mice described in the sections [Aging mice] and [Young mice] below are used to carry out analysis according to the methods described in the sections [Method 1] to [Method 3] below.

[Aging mice]

Water containing three compounds (racemic form of MAS, S(+) form of MAS, or R(-) form of MA5) is orally administered to aging mice (92 weeks) (5 mice) at a dose of 50 mg/kg body weight/day each for 9 weeks. In addition, as a control, water is orally administered to aging mice (6 mice) for 9 weeks.

[Young mice]

Water containing three compounds (racemic form of MAS, S(+) form of MAS, or R(-) form of MAS) is orally administered to young mice (4 months) (6 mice) at a dose of 10 mg/kg body weight/day (low dose) or 50 mg/kg body weight (high dose) each for 9 weeks. In addition, as a control, water is orally administered to young mice (6 mice) for 9 weeks.

[Method 1: Treadmill test]

[1] A treadmill apparatus for mice (MK-690, manufactured by Muromachi Kikai Co., Ltd.; hereinafter the same applies) is used to provide training for a treadmill test. Aging mice undergo a total of 3 days of training: day 1; twice × at a speed of 8 m/min for 10 minutes, day 2; twice × at a speed of 10 m/min for 10 minutes, and day 3; twice × at a speed of 12 m/min for 10 minutes. On the other hand, young mice undergo a total of 5 days of training: day 1; twice × at a speed of 8 m/min for 10 minutes, day 2; twice × at a speed of 9 m/min for 10 minutes, day 3; twice × at a speed of 10 m/min for 10 minutes; day 4; twice × at a speed of 11 m/min for 10 minutes, and day 5; twice × at a speed of 12 m/min for 10 minutes.

[2] The treadmill apparatus for mice is used to carry out the actual treadmill test for up to 2 hours under the following conditions. The test is started with a speed of 5 m/min, and the speed is increased by 5 m/min every 3 minutes, finally to a speed of 28 m/min (that is, 5 m/min → 10 m/min → 15 m/min → 20 m/min → 25 m/min → 28 m/min).

[3] When the mouse does not move forward even when the tail is stimulated with a brush and does not move on an energized rod for 10 seconds or more, it is determined that the mouse has exhausted itself. When the mouse runs for more than 2 hours, the test is discontinued, and the time is recorded as 2 hours.

[Method 2: Inverse grating suspension test]

A mouse inverse grating suspension test is carried out by using a wire mesh that has a diameter of 37 cm and that is 8 mm square. A 12 cm plastic wall is constructed around the wire mesh for the purpose of preventing a mouse from escaping. A mouse is placed on the wire mesh for 1 minute for acclimatization. The wire mesh is turned upside down with the mouse in the center of the wire mesh, and the time (minutes) until the mouse falls is measured. The wire mesh is held at a height of 60 cm from the ground. In order to prevent the mouse from being injured when the mouse falls, bedding for raising is laid on the ground. When the mouse falls in less than 30 seconds, it is determined that the fall is an accidental fall, and an additional measurement is carried out until the mouse falls in 30 seconds or more. The measurement is carried out twice on another day, and the average value thereof is used. In addition, the body weight of the mouse is measured, and the "Hanging Impulse Score" (hanging time [minutes] × body weight [minutes]) is calculated.

[Method 3: Grip strength]

Mouse grip strength is measured by using a mouse grip strength measuring apparatus (GPM-101, manufactured by Melquest Ltd.). The grip strength of the front legs is measured by using a grip for the front legs, and the grip strength of the four legs is measured by setting a wire mesh for the four legs. The measurement is started by measuring the grip strength of the front legs, which is measured in the lateral direction and the longitudinal direction. For the measurement in the lateral direction, the mouse is pulled in the horizontal direction with both front legs of the mouse holding the grip until the paws are released. For the measurement in the longitudinal direction, the mouse is pulled in the vertical direction with both front legs of the mouse holding the grip until the paws are released. The maximum value of 5 consecutive measurements is measured. For the measurement for the four legs, the tail is pulled horizontally with the four legs of the mouse placed on the wire mesh until the paws are released, and the maximum value of 5 consecutive runs is measured.

14. Nampt binding test

The binding of the S(+) form of MAS and the R(-) form of MAS to nicotinamide phosphoribosyltransferase (Nampt), which is a key enzyme of the mammalian $NAD^+$ synthesis system, was measured by using Octet K2.

A 0.01% DDM PBS solution was prepared as a measurement buffer. 1 μL of biotin Nampt was added to 1000 μL of the measurement buffer. This was diluted 2-fold to obtain a 0.4 ng/μL ligand solution. A DMSO solution of a 100 mM MAS S(+) form was diluted from 300 μM to 1000 μM, a DMSO solution of a 100 mM MAS R(-) form was diluted from 500 μM to 1200 μM, and the diluted solutions were each used as an analyte solution. 200 μL each of the measurement buffer, a 1 μM biotin solution, a ligand solution, a 100 μM biocytin solution, and the analyte solution were added to a 96-well plate, and the plate was set in Octet K2 to carry out the measurement. Data were analyzed by using the accompanying software. Kd values were represented graphically with Prism 9 by using the concentrations of the analyte solution and the numerical values of Req (nm) calculated by the analysis.

It was confirmed that the S(+) form of MAS binds to Nampt, but the R(-) form does not bind to Nampt (Figure 15). It is known that Nampt is an enzyme that controls the synthesis of $NAD^+$, and thus the above result is consistent with the result that the S(+) form of MAS increases the $NAD^+$ level.

15. Effect of suppressing DNA damage caused by camptothecin

Rat fetal cardiomyoblast cells H9c2 were maintained and cultured in a DMEM medium (10% FBS, penicillin at 100 units/mL, streptomycin at 100 μg/mL). Camptothecin (CPT) at a final concentration of 10 μM, CPT at a final concentration of 10 μM, and MAS (racemic form, S(+) form, or R(-) form) at a final concentration of 30 μM, or DMSO (control) were added. The cells were cultured for 24 hours, then the medium was removed, and yH2AX (phosphorylated H2AX), which is an indicator for DNA damage, was detected by immunostaining. The immunostaining was carried out by using a mouse anti-γH2AX antibody (Thermo Fisher) as a primary antibody and a CF568-labeled goat anti-mouse IgG antibody (Biotium Inc.) as a secondary antibody.

As a result of the immunostaining, a decrease in the fluorescence intensity of phosphorylated H2AX was observed only in the samples to which the S(+) form of MAS was added (Figure 16), confirming that the S(+) form of MAS has the effect of suppressing DNA damage caused by CPT. The above effect was not observed for the R(-) form. It is known that an increase in $NAD^+$ level has a DNA repair effect, and therefore, the above results suggest that the S(+) form of MAS can suppress DNA damage caused by CPT through increasing the $NAD^+$ level.

16. Physical properties of S(+) form of MAS

According to comparison of physical properties of the S(+) form of MA5 with those of the racemic form, the S(+)

form of MA5 is easily soluble in acetone, acetonitrile, ethyl acetate, chloroform, dichloromethane, methanol, ethanol, and isopropanol. In addition, the S(+) form of MA5 is slightly soluble in a 50% acetonitrile aqueous solution, but sparingly soluble in a 50% methanol aqueous solution. On the other hand, the racemic form of MA5 is slightly soluble in a chloroform/methanol mixed solvent, a dichloromethane/methanol mixed solvent, and acetone, but sparingly soluble in ethyl acetate, chloroform, dichloromethane, and methanol.

In addition, the S(+) form of MA5 undergoes coloration and degradation under a light, acid, or alkali condition. Specifically, a dissolution solution of the S(+) form of MA5 in ethanol, isopropanol, acetone, acetonitrile, ethyl acetate, a 50% acetonitrile aqueous solution, or a 0.1% formic acid-containing acetonitrile solution is colorless and transparent, and does not become colored even after refrigerated for about a week. However, a dissolution solution obtained by dissolving the S(+) form of MA5 in methanol, dichloromethane, chloroform, a 50% methanol aqueous solution, or a 0.1% formic acid-containing methanol solution was colorless and transparent on the day of preparation, but colored pale yellow when refrigerated for about a week. Furthermore, a dissolution solution obtained by dissolving the S(+) form of MA5 in a mixed solvent of an alcohol (methanol, ethanol, or isopropanol) and chloroform was colored yellow the day after preparation.

A halogenated solvent generates hydrogen chloride as a slight amount of a degradation product, and thus, for example, chloroform includes ethanol as a stabilizer in an amount of 0.4 to 0.8%. It has been confirmed that a mixed solvent of an alcohol and chloroform promotes coloration and degradation, and it is considered that the presence of a slight amount of an acid and the presence of an alcohol may contribute to the degradation.

When a dissolution solution of the S(+) form of MA5 was spotted on silica gel TLC plate and developed after some time, a degradation product appeared, but when the dissolution solution was developed immediately, no degradation product was generated. A degradation product was not easily generated in an ethyl acetate dissolution solution, whereas a large amount of a degradation product was generated in an alcohol-chloroform dissolution solution.

TLC is under a slightly acidic condition, which may promote the degradation. Furthermore, a combination of an alcohol (short chain alcohol) and chloroform tends to promote degradation more.

17. Generation of salt of S(+) form of MA5

17-1 Sodium salt

The S(+) form of MA5 (0.99 g, 3.01 mmol) was placed in a 100 mL round bottom flask, followed by addition of ultrapure water (23 mL) and 2 mol/L aqueous sodium hydroxide solution (1.21 mL, 0.8 eq). The mixture was stirred at room temperature for 1.5 hours, resulting in dissolution. The reaction mixture was filtered to remove insoluble matter. A crude sodium salt of the S(+) form of MAS was distilled out of the filtrate at reduced pressure. The crude salt was redissolved in ultrapure water. The mixture was concentrated with an evaporator to obtain a supersaturated solution, which was left to stand at 4°C to obtain light yellow to white crystals of a sodium salt of the S(+) form of MAS (159.6 mg, 0.454 mmol, yield 32%).

$^1$H NMR (DMSO-$d_6$): δ 10.63 (s, 1H), 7.88-7.84 (m, 1H), 7.66 (d, J = 7.9 Hz, 1H), 7.35-7.32 (m, 1H), 7.26 (d, J = 8.1 Hz, 1H), 7.19-7.16 (m, 1H), 7.00-6.97 (m, 2H), 6.89-6.87 (m, 1H), 4.03 (dd, J = 9.5, 5.2 Hz, 1H), 3.80-3.76 (m, 1H), 2.91-2.87 (m, 1H); LRMS m/z $[M-H]^-$ 317.90.

A sodium salt of the R(-) form of MAS and a sodium salt of the racemic form of MAS were also synthesized in the same method.

17-2 Calcium salt

The S(+) form of MAS (295 mg, 0.896 mmol) was weighed in a 20 mL round bottom flask, followed by addition of 2-propanol (6 mL), resulting in dissolution. Then, 1 mg/mL calcium hydroxide solution (45 mL, 0.68 eq) was added to this solution, followed by mixing. The solvent was distilled off from the reaction solution at reduced pressure to obtain a crude calcium salt of the S(+) form of MA5. The crude salt was redissolved in ethanol (10 mL). Ultrapure water (50 mL) was added to the solution while the solution was warmed at 40°C. The solution was concentrated with an evaporator to obtain a supersaturated solution, which was left to stand at room temperature to obtain yellow to white crystals of a calcium salt of the S(+) form of MAS (170.0 mg, 0.244 mmol, yield 54%).

$^1$H NMR (DMSO-$d_6$): δ 10.68 (s, 1H), 7.87 (q, J = 7.9 Hz, 1H), 7.65 (d, J = 7.9 Hz, 1H), 7.37-7.34 (m, 1H), 7.27 (d, J = 8.1 Hz, 1H), 7.19-7.17 (m, 1H), 7.06 (s, 1H), 7.01-6.99 (m, 1H), 6.92-6.89 (m, 1H), 4.09 (d, J = 1.4 Hz, 1H) 3.83-3.79 (m, 1H), 3.01-2.98 (m, 1H); LRMS m/z $[M-H]^-$ 317.89.

A calcium salt of the R(-) form of MAS and a calcium salt of the racemic form of MAS are also synthesized in the same method.

17-3 Potassium salt

The S(+) form of MAS (0.214 g, 0.650 mmol) was weighed in a 100 mL round bottom flask, followed by addition of ultrapure water (4 mL) and 2 mol/L potassium hydroxide (0.28 mL, 0.86 eq). The mixture was stirred at room temperature for 1 hour, resulting in dissolution. Insoluble matter was filtered off from the reaction solution to obtain filtrate. The solvent was distilled off from the filtrate at reduced pressure to obtain a crude potassium salt of the S(+) form of MA5. The crude salt was redissolved in ultrapure water. The mixture was lyophilized to obtain a potassium salt of the S(+) form of MAS (123.0 mg, 0.335 mmol, yield 52%).
$^1$H NMR (DMSO-$d_6$): δ 10.61 (s, 1H), 7.87-7.83 (m, 1H), 7.63 (d, J = 7.9 Hz, 1H), 7.34-7.30 (m, 1H), 7.25 (d, J = 8.1 Hz, 1H), 7.18-7.15 (m, 1H), 6.99-6.97 (m, 2H), 6.88-6.86 (m, 1H), 3.97 (dd, J = 9.3, 5.3 Hz, 1H), 3.76-3.71 (m, 1H), 2.89-2.85 (m, 1H); LRMS m/z $[M-H]^-$ 317.91.
A potassium salt of the racemic form of MAS was also synthesized in the same method. A potassium salt of the R(-) form of MAS is also synthesized in the same method.

18. Chiral separation analysis

It was confirmed that the salts prepared from the enantiomers of MAS by the method described in the section 17 did not undergo chiral inversion (conversion).

18-1 Method

As an ionization unit, an apparatus in which NANOSPACE SI-2 (manufactured by OSAKA SODA CO., LTD.) as a high-performance liquid chromatograph was connected to a triple quadrupole mass spectrometer (TSQ Quantum Ultra, manufactured by Thermo Fisher Scientific Inc.) equipped with an ESI probe was used. The S(+) form of MAS and the R(-) form of MAS, which are substances to be analyzed, were subjected to quantitative analysis by the internal standard method using the racemic form of MA5-$d_5$, which is a stable isotope. CHIRALPAK AD-3R (150 × 2.1 mm i.d., 3 μm particle size, manufactured by Daicel Corporation) was used as the analytical column, and the column oven temperature was 35°C. Then, 10% acetonitrile containing 0.1% formic acid was used as the mobile phase solution A, acetonitrile was used as the mobile phase solution B, the flow rate was 200 μL/min, and linear gradient elution was performed (50% solution B (0.0 to 4.0 minutes), 90% solution B (4.1 to 6.9 minutes), 100% solution B (7.0 to 8.0 minutes), 50% solution B (8.1 to 10.5 minutes)). The sample injection volume was set to 2 μL, and 50% methanol was selected as the wash solution. The retention time of the S(+) forms of MAS and MA5-ds was 4.3 minutes, and the retention time of the R(-) forms of MA5 and MA5-$d_5$ was 5.4 minutes.
Detection was performed in negative ion mode, with a spray voltage set to 3.0 kV, a vaporizer temperature set to 350°C, and a capillary temperature set to 250°C. The precursor ion>product ion SRM channels of the S(+) forms of MAS and the R(-) forms of MAS were set to "m/z 328.1>116.0", and the precursor ion>product ion SRM channels of the S(+) forms of MA5-$d_5$ and the R(-) forms of MAS were set to "m/z 333.1>121.0". The collision energy for generating a product ion was set to 15 eV. Waveform analysis and quantitative calculation by the internal standard method were carried out by using Xcalibur 3.0 (manufactured by Thermo Fisher Scientific Inc.).

18-2 Results

The optical purities of the salts prepared from the enantiomers of MAS by the methods described in the section 17 were confirmed by the above-mentioned method, and it was found that the amounts of the optical isomers were at or below the detection limit, indicating that chiral conversion did not occur.

19. Test of water solubility of MAS

The water solubilities of the racemic form and the enantiomers of MAS and the salts thereof were analyzed according to the following method.

19-1 Method

Microtubes were charged with the racemic form and the S(+) form of MAS, and the sodium salts, the calcium salts, or the potassium salts thereof and shaken under supersaturation conditions (20 mg/20 μL ultrapure water) for 30 minutes, resulting in dissolution by suspension. Each solution was filtered through a filter of 0.22 μm to obtain a saturated solution, which was 1-, 100-, and 1000-fold diluted with ultrapure water to prepare a measurement sample. The prepared measurement samples were measured by LC-MS/MS (liquid chromatography/tandem mass spectrometry) to calculate

the water solubility of MA5. The analysis conditions for LC-MS/MS are as described below.

As an ionization unit, an apparatus in which ACQUITY UPLC (manufactured by Nihon Waters K.K.) as a high-performance liquid chromatograph was connected to a triple quadrupole mass spectrometer (TSQ Endura, manufactured by Thermo Fisher Scientific Inc.) equipped with an ESI probe was used. MA5, which is a substance to be analyzed, was subjected to quantitative analysis by the internal standard method using MA5-$d_5$, which is a stable isotope. ACQUITY UPLC BEH Phenyl (50 × 2.1 mm i.d., 1.7 μm particle size, manufactured by Nihon Waters K.K.) was used as the analytical column, and the column oven temperature was 50°C. Then, 10 mM $CH_3COONH_4/H_2O$ was used as the mobile phase solution A, methanol was used as the mobile phase solution B, the flow rate was 500 μL/min, and linear gradient elution was performed (10% solution B (0.0 to 1.5 minutes), 20% solution B (1.5 to 2.5 minutes), 20 to 35% solution B (2.5 to 7.5 minutes), 35 to 100% solution B (7.5 to 8.5 minutes), 100% solution B (8.5 to 9.2 minutes), 10% solution B (9.2 to 9.7 minutes)). The sample injection volume was set to 5 μL, 50% methanol was selected as the purge wash solution, and 50% acetonitrile was selected as the needle wash solution. The retention time of MAS was 7.2 minutes, and the retention time of MA5-$d_5$ was 7.1 minutes.

For the MS conditions, detection was performed in negative ion mode, with the spray voltage set to 3.0 kV, the vaporizer temperature set to 350°C, and the ion transfer tube temperature set to 250°C. The precursor ion>product ion SRM channel of MAS was set to "m/z 328.1>116.0", and the precursor ion>product ion SRM channel of MA5-$d_5$ was set to "m/z 333.1>121.0". The collision energy for generating a product ion was set to 15 eV. Waveform analysis and quantitative calculation by the internal standard method were carried out by using Xcalibur 3.0 (manufactured by Thermo Fisher Scientific Inc.).

19-2 Results

The water solubilities calculated from the results of the quantification and the classification of solubilities according to the General Notices of the Japanese Pharmacopoeia are as follows, and MAS was formed into a sodium salt or a potassium salt, so that the solubility was greatly improved.

Racemic form of MA5: 0.00537 g/L, scarcely soluble
Sodium salt of racemic form of MA5: 65 g/L, slightly easily soluble
Potassium salt of racemic form of MA5: 216 g/L, easily soluble
S(+) form of MA5: 0.0549 g/L, scarcely soluble
Sodium salt of S(+) form of MA5: 251 g/L, easily soluble
Calcium salt of S(+) form of MA5: 2.95 g/L, scarcely soluble
Potassium salt of S(+) form of MA5: 607 g/L, easily soluble

20. Light exposure stability of MA5

The light exposure stabilities of the racemic form and the enantiomers of MA5 and the salts thereof were analyzed according to the following method.

20-1 Method

Microtubes were charged with the racemic form and the S(+) form of MA5, and the sodium salts or the calcium salts thereof, and the microtubes were lidded and left to stand under a white fluorescent lamp radiating light at room temperature for 14 days. Each specimen was dissolved at 1 mg/mL in acetonitrile containing 0.2% acetic acid to be prepared as a measurement sample. The prepared measurement sample was measured by LC-UV (liquid chromatography/ultraviolet detection), and the light exposure stability of MA5 was evaluated from the chromatogram area. The LC-UV analysis conditions are as follows.

NANOSPACE SI-2 (manufactured by OSAKA SODA CO., LTD.) was used as a high-performance liquid chromatograph. CAPCELL PAK C18 ACR (150 × 1.5 mm i.d., 3 μm particle size, manufactured by OSAKA SODA CO., LTD) was used as an analytical column, and the column oven temperature was 40°C. $H_2O$ was used as the mobile phase solution A, acetonitrile was used as the mobile phase solution B, the flow rate was 180 μL/min, and linear gradient elution was performed (20 to 80% solution B (0.0 to 10.0 minutes), 100% solution B (10.1 to 15.0 minutes), 20% solution B (15.1 to 20.0 minutes)). The sample injection volume was set to 3 μL, and 50% methanol was selected as the wash solution. The retention time of MAS was 8.0 minutes.

20-2 Results

In the method for evaluating the stability, the stability was evaluated using the ratio of the peak area of MAS to the

total peak area of the chromatogram obtained. The results of comparison with the racemic form of MAS, the S(+) form of MAS, and the sodium salts or the calcium salts thereof not exposed to light as controls are as follows. The peak areas of impurities considered to be decomposition products increased with respect to the racemic form of MAS, the S(+) form of MAS, and the sodium salt of the racemic form of MA5. Meanwhile, the S(+) form of MAS was formed into a sodium salt or a calcium salt, so that the photostability was improved, and little decomposition products were observed.

[Table 7]

| | Control | Exposed to light |
|---|---|---|
| MA5 racemic form | 99.76±0.01% | 97.03±0.05 % |
| MA5 racemic form Na salt | 99.91±0.01% | 98.11±0.30% |
| MA5 S(+) | 99.94±0.00% | 95.71±0.13% |
| MA5 S(+) Na salt | 99.93±0.01% | 99.59±0.08% |
| MA5 S(+) Ca salt | 99.94%(N=1) | 99.92±0.00% |

**Industrial Applicability**

The present invention contributes to prevention or treatment of a symptom or a disease such as a symptom or a disease associated with aging, a metabolic disease, or a mitochondrial disease, and to the extension of the lifespan.

All publications, patents, and patent applications cited herein are incorporated herein by reference in their entirety.

**Claims**

1. A pharmaceutical composition comprising a compound represented by the following formula (A-1), substantially free of an R(-) form, or a physiologically acceptable salt thereof as an active ingredient.

[Chemical Formula 1]

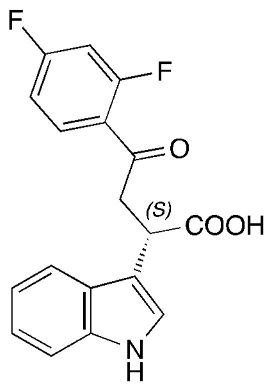

(A-1)

2. The pharmaceutical composition according to claim 1, wherein the pharmaceutical composition is used to increase an $NAD^+$ level in a subject.

3. The pharmaceutical composition according to claim 1, wherein the pharmaceutical composition is for treatment or prevention of a mitochondrial disease.

4. The pharmaceutical composition according to claim 1, wherein the pharmaceutical composition is for treatment or prevention of a symptom or a disease associated with aging.

5. The pharmaceutical composition according to claim 4, wherein the symptom or the disease associated with aging is hearing loss.

6. The pharmaceutical composition according to claim 1, wherein an enantiomeric excess of an S(+) form is at least 97%.

7. The pharmaceutical composition according to any one of claims 1 to 6, wherein the compound represented by formula (A-1) or the physiologically acceptable salt thereof is a sodium salt, a potassium salt, or a calcium salt, preferably a sodium salt or a potassium salt, and more preferably a sodium salt, of the compound represented by formula (A-1).

8. A food or drink composition comprising a compound represented by the following formula (A-1), substantially free of an R(-) form, or a physiologically acceptable salt thereof as an active ingredient.

[Chemical Formula 2]

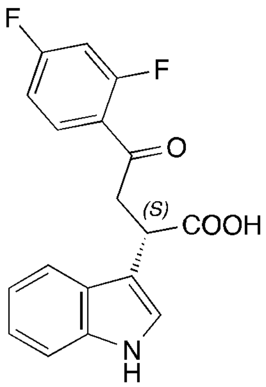

(A-1)

9. The food or drink composition according to claim 8, wherein the food or drink composition is used to increase an $NAD^+$ level in a subject.

10. The food or drink composition according to claim 8, wherein the food or drink composition is for a subject in need of treating or preventing a mitochondrial disease.

11. The food or drink composition according to claim 8, wherein the food or drink composition is for a subject in need of treating or preventing a symptom or a disease associated with aging.

12. The food or drink composition according to claim 11, wherein the symptom or the disease associated with aging is hearing loss.

13. The food or drink composition according to claim 8, wherein an enantiomeric excess of an S(+) form is at least 97%.

14. The food or drink composition according to any one of claims 8 to 13, wherein the compound represented by formula (A-1) or the physiologically acceptable salt thereof is a sodium salt, a potassium salt, or a calcium salt, preferably a sodium salt or a potassium salt, and more preferably a sodium salt, of the compound represented by formula (A-1).

[FIG.1 ]

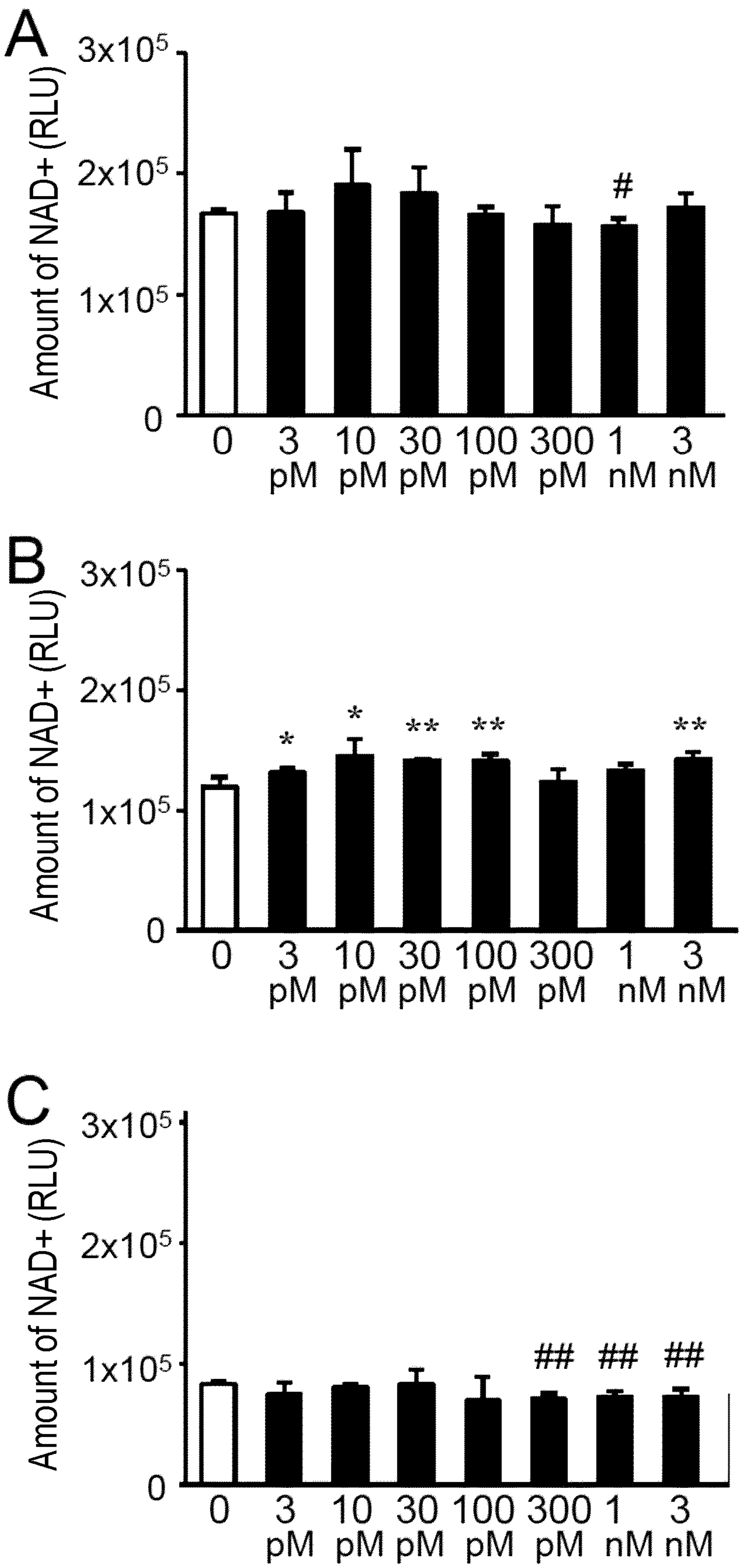
A
Amount of NAD+ (RLU)
3x10^5
2x10^5
1x10^5
0
#
0
3 pM
10 pM
30 pM
100 pM
300 pM
1 nM
3 nM
B
Amount of NAD+ (RLU)
3x10^5
2x10^5
1x10^5
0
*
*
**
**
**
0
3 pM
10 pM
30 pM
100 pM
300 pM
1 nM
3 nM
C
Amount of NAD+ (RLU)
3x10^5
2x10^5
1x10^5
0
##
##
##
0
3 pM
10 pM
30 pM
100 pM
300 pM
1 nM
3 nM

[FIG.2 ]

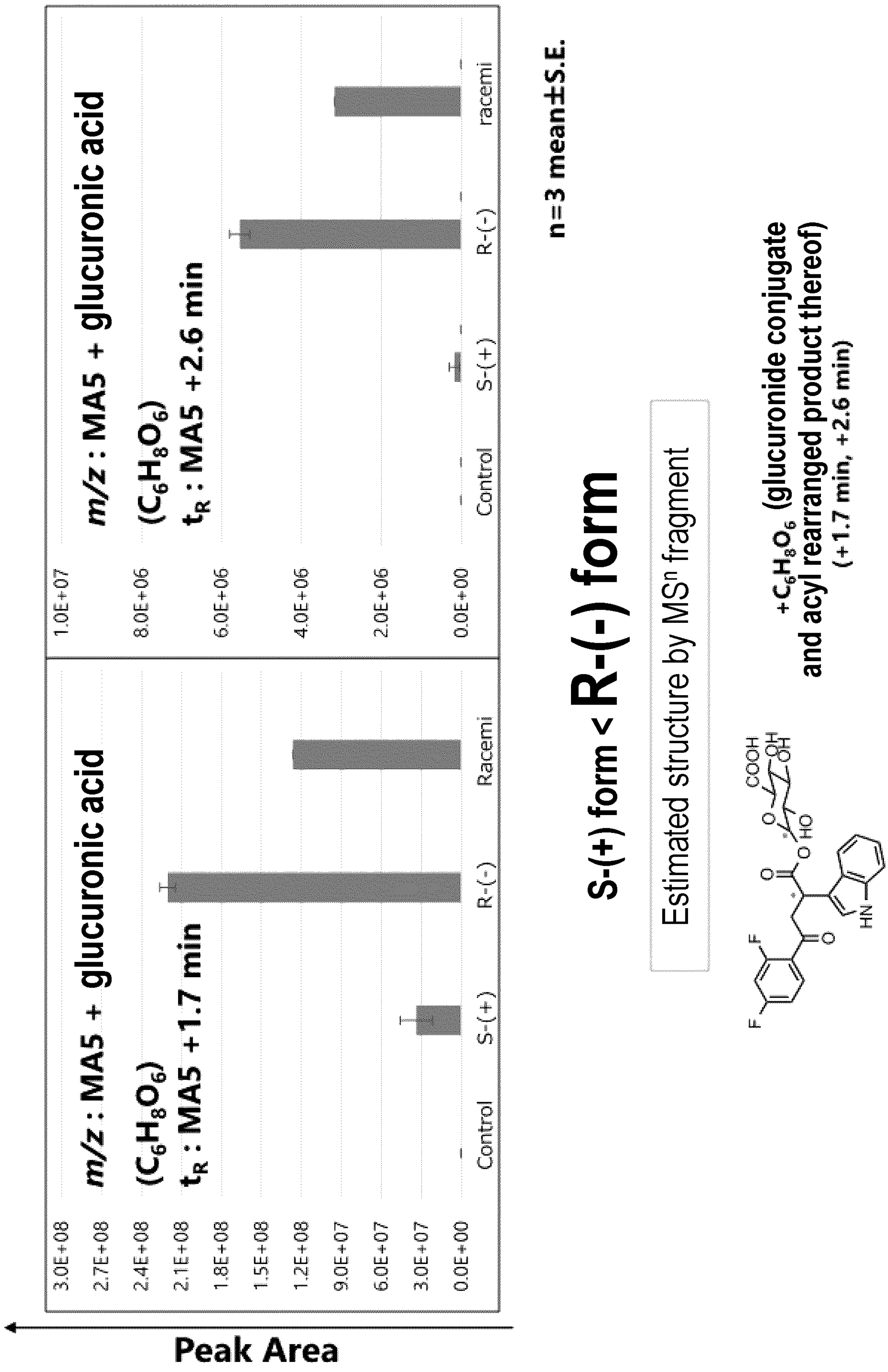
Peak Area
m/z : MA5 + glucuronic acid
($C_6H_8O_6$)
$t_R$ : MA5 +1.7 min
3.0E+08
2.7E+08
2.4E+08
2.1E+08
1.8E+08
1.5E+08
1.2E+08
9.0E+07
6.0E+07
3.0E+07
0.0E+00
Control
S-(+)
R-(-)
Racemi
m/z : MA5 + glucuronic acid
($C_6H_8O_6$)
$t_R$ : MA5 +2.6 min
1.0E+07
8.0E+06
6.0E+06
4.0E+06
2.0E+06
0.0E+00
Control
S-(+)
R-(-)
racemi
n=3 mean±S.E.
S-(+) form < R-(-) form
Estimated structure by $MS^n$ fragment
$+C_6H_8O_6$ (glucuronide conjugate
and acyl rearranged product thereof)
(+1.7 min, +2.6 min)

[FIG.3 ]

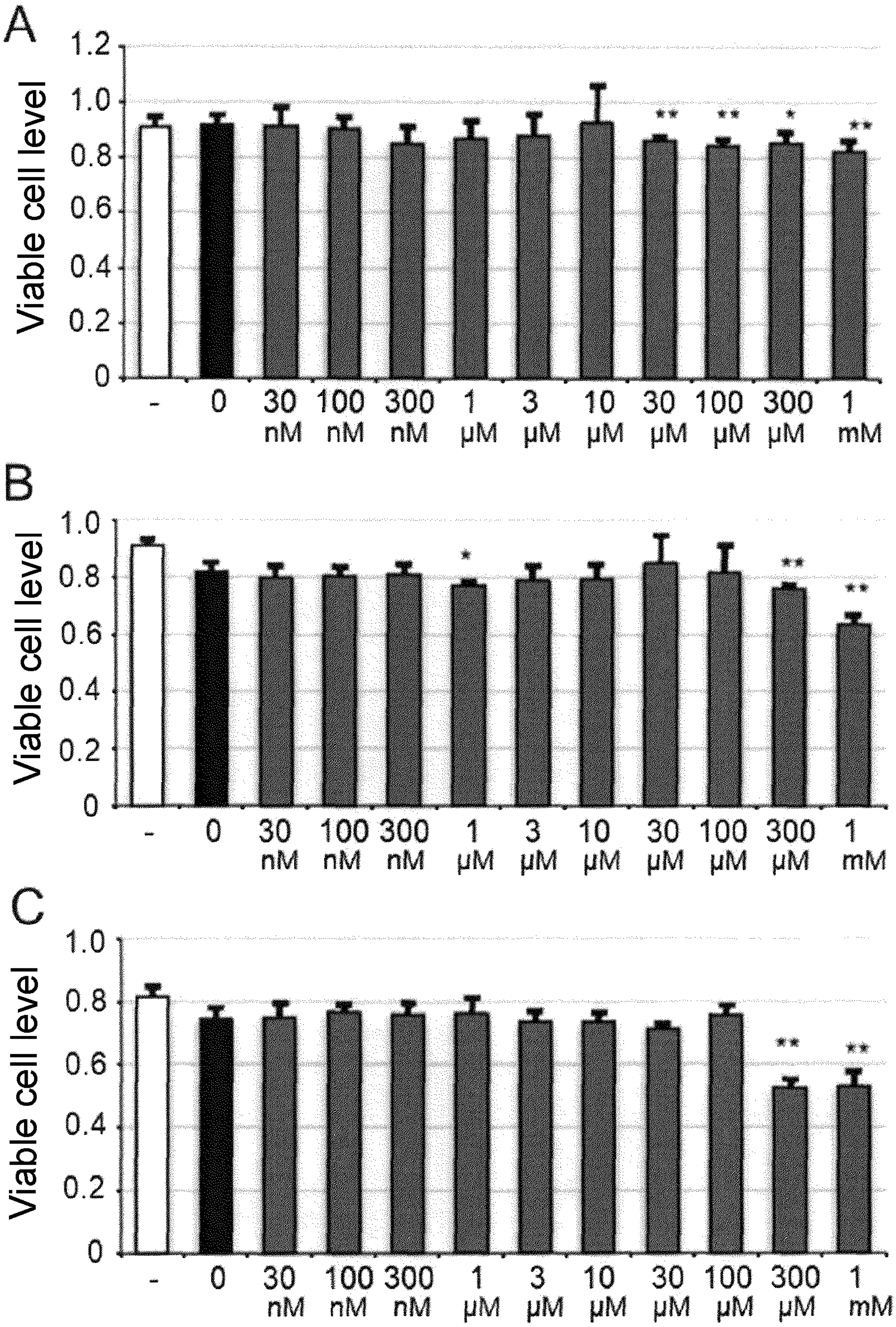
A
Viable cell level
1.2
1.0
0.8
0.6
0.4
0.2
0
- 0 30 nM 100 nM 300 nM 1 μM 3 μM 10 μM 30 μM 100 μM 300 μM 1 mM
B
Viable cell level
1.0
0.8
0.6
0.4
0.2
0
- 0 30 nM 100 nM 300 nM 1 μM 3 μM 10 μM 30 μM 100 μM 300 μM 1 mM
C
Viable cell level
1.0
0.8
0.6
0.4
0.2
0
- 0 30 nM 100 nM 300 nM 1 μM 3 μM 10 μM 30 μM 100 μM 300 μM 1 mM

[FIG.4 ]

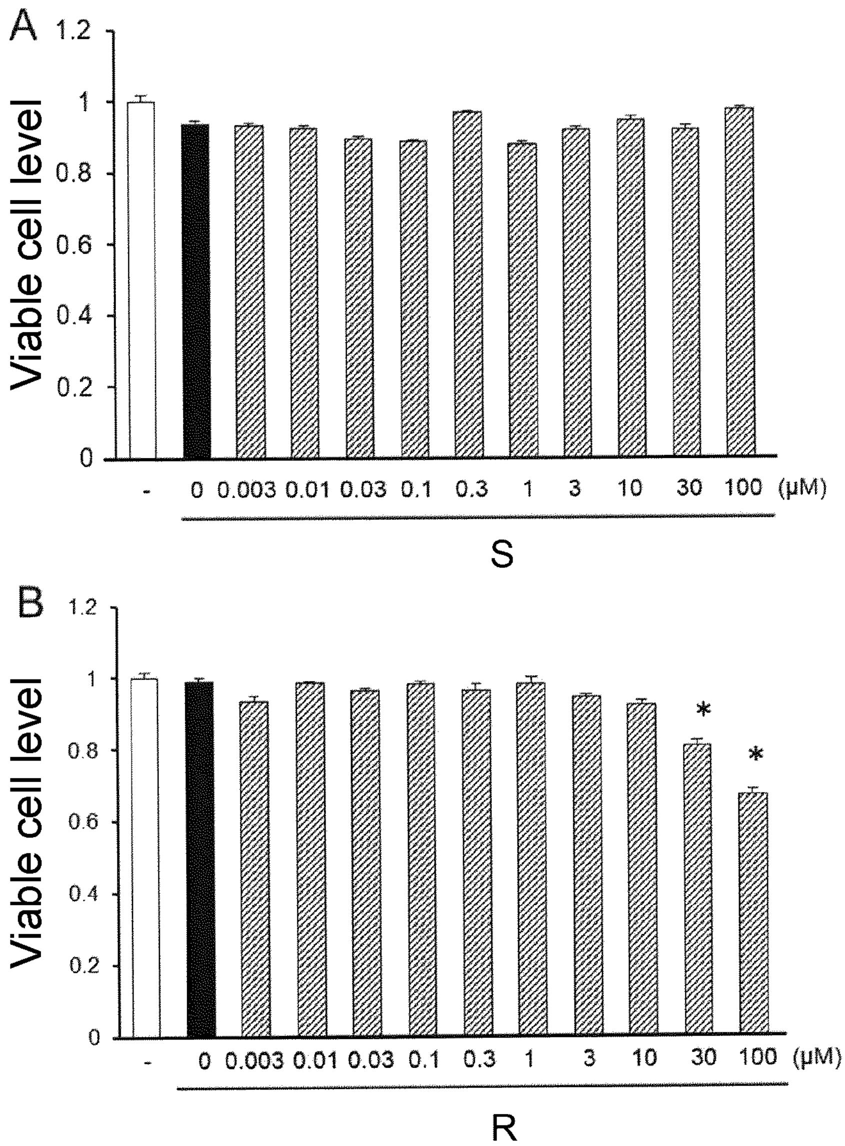
A
1.2
1
0.8
0.6
0.4
0.2
0
Viable cell level
- 0 0.003 0.01 0.03 0.1 0.3 1 3 10 30 100 (μM)
S
B
1.2
1
0.8
0.6
0.4
0.2
0
Viable cell level
*
*
- 0 0.003 0.01 0.03 0.1 0.3 1 3 10 30 100 (μM)
R

[FIG.5 ]

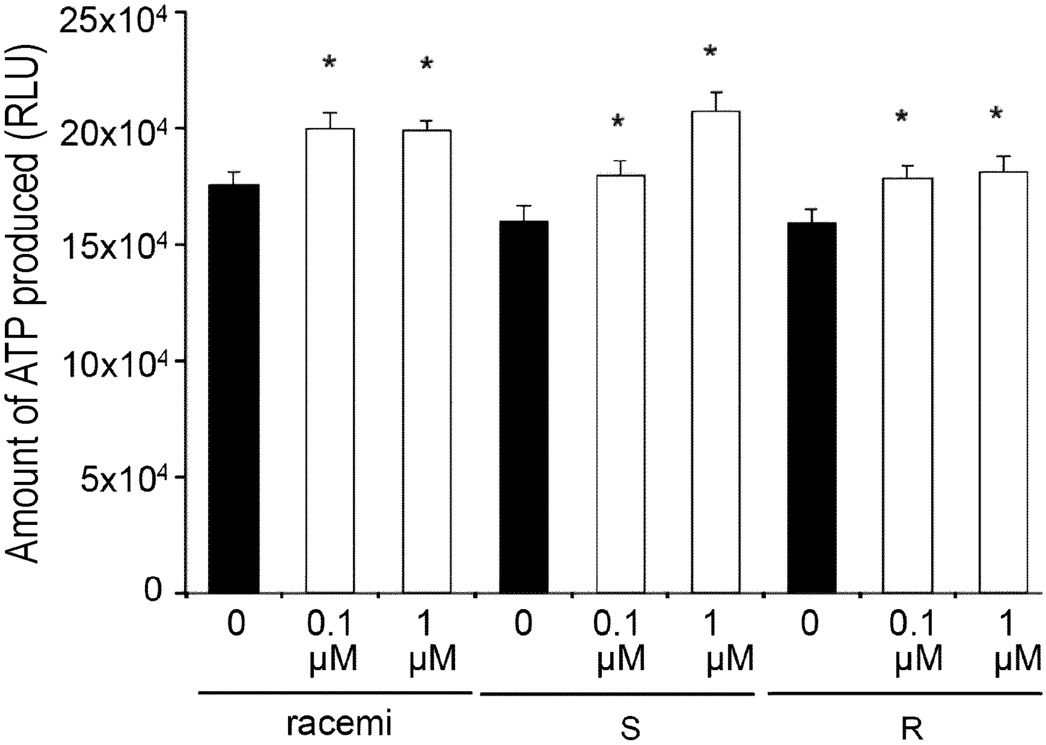
25x10⁴
20x10⁴
15x10⁴
10x10⁴
5x10⁴
0
Amount of ATP produced (RLU)
*
0
0.1 μM
1 μM
racemi
S
R

[FIG.6 ]

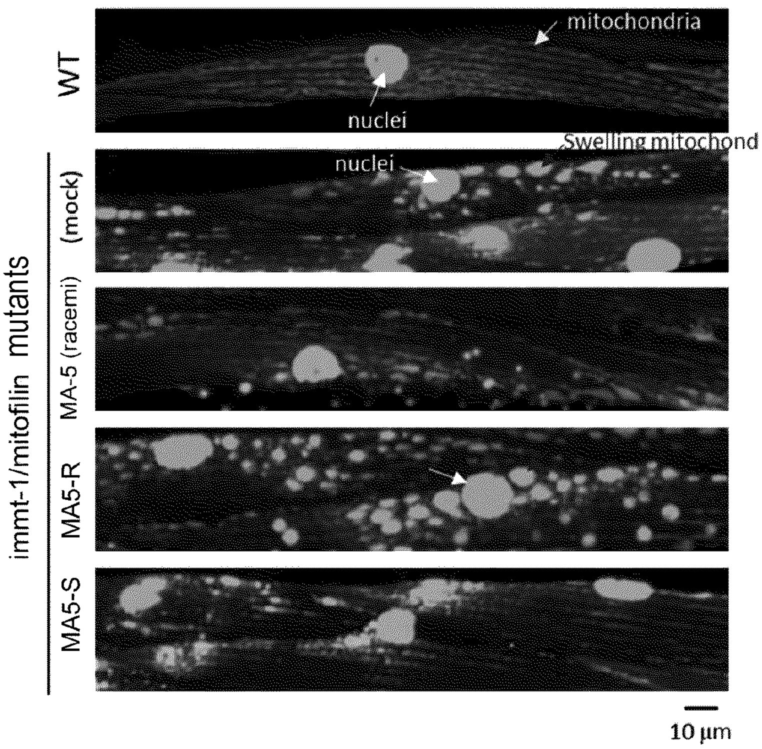
WT
mitochondria
nuclei
Swelling mitochond
immt-1/mitofilin mutants
(mock)
MA-5 (racemi)
MA5-R
MA5-S
10 μm

[FIG.7 ]

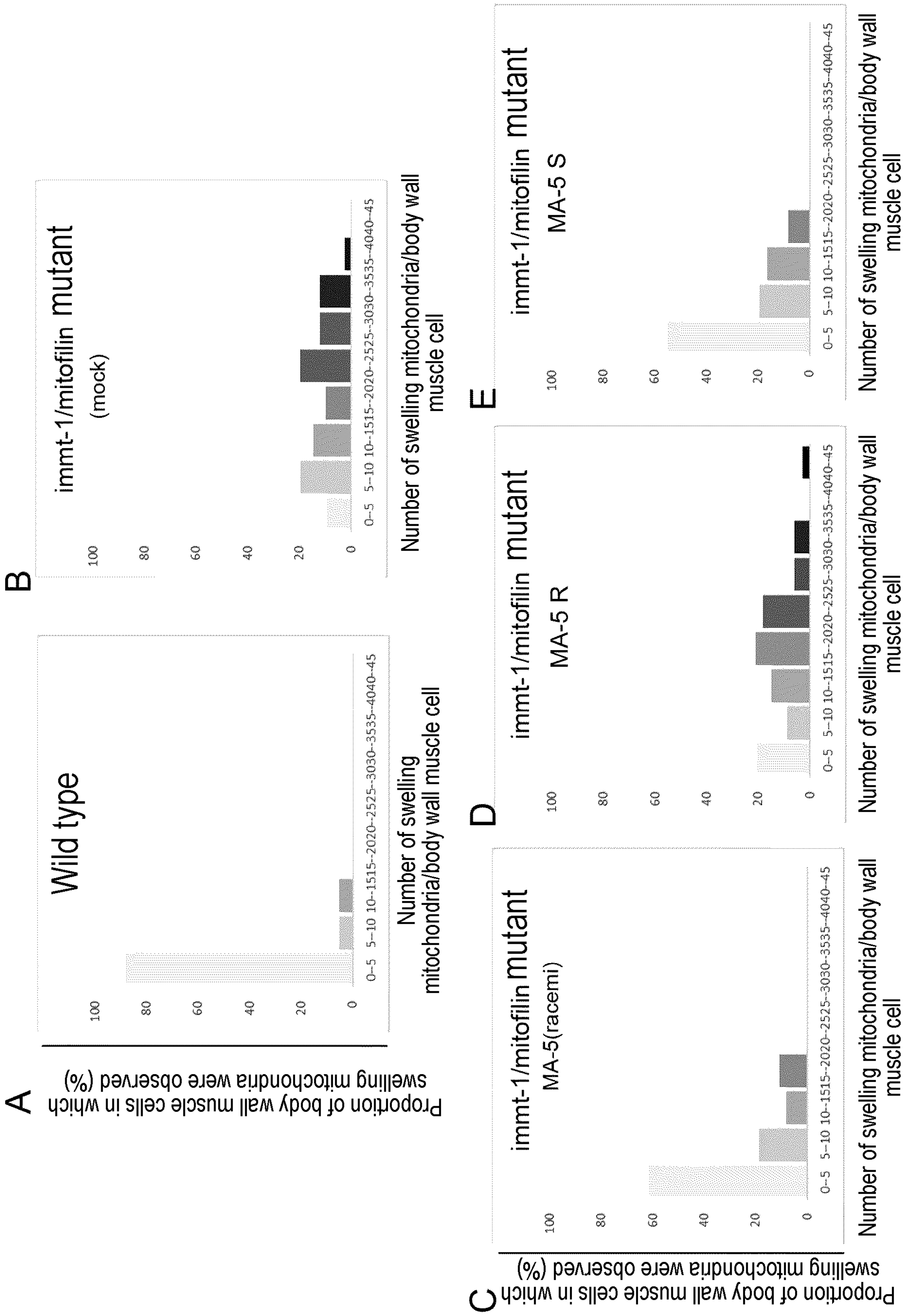
A
Wild type
B
immt-1/mitofilin mutant
(mock)
C
immt-1/mitofilin mutant
MA-5(racemi)
D
immt-1/mitofilin mutant
MA-5 R
E
immt-1/mitofilin mutant
MA-5 S
Proportion of body wall muscle cells in which swelling mitochondria were observed (%)
Number of swelling mitochondria/body wall muscle cell
0–5 5–10 10–15 15–20 20–25 25–30 30–35 35–40 40–45
0 20 40 60 80 100

[FIG.8 ]

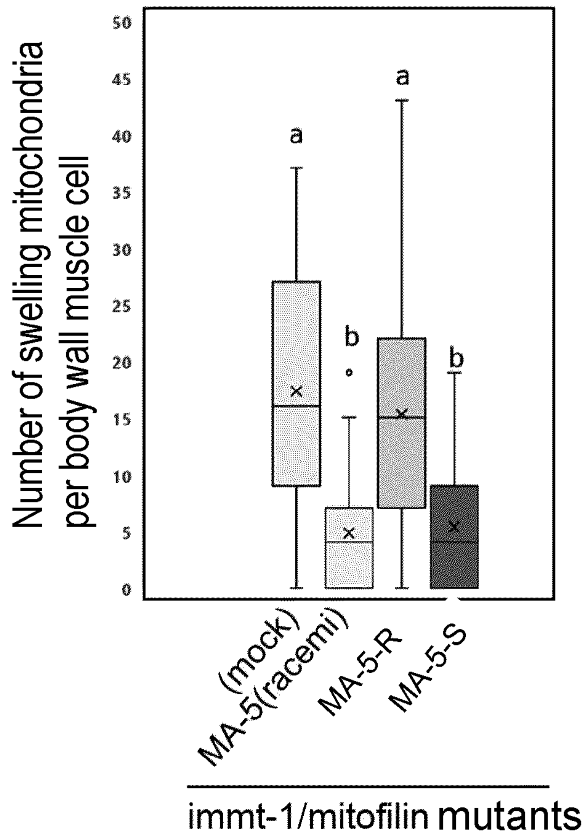
Number of swelling mitochondria
per body wall muscle cell
50
45
40
35
30
25
20
15
10
5
0
a
b
a
b
(mock)
MA-5(racemi)
MA-5-R
MA-5-S
immt-1/mitofilin mutants

[FIG.9 ]

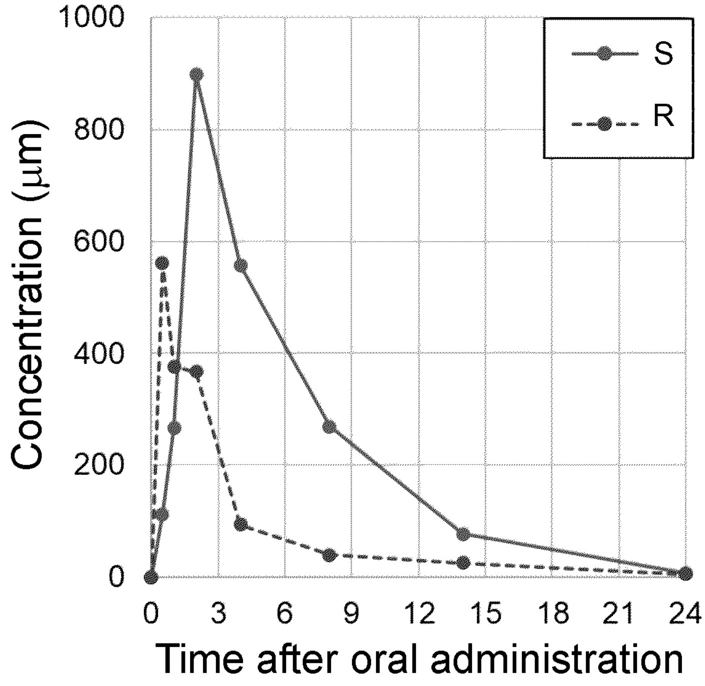
1000
800
600
400
200
0
Concentration (μm)
0
3
6
9
12
15
18
21
24
Time after oral administration
S
R

[FIG.10 ]

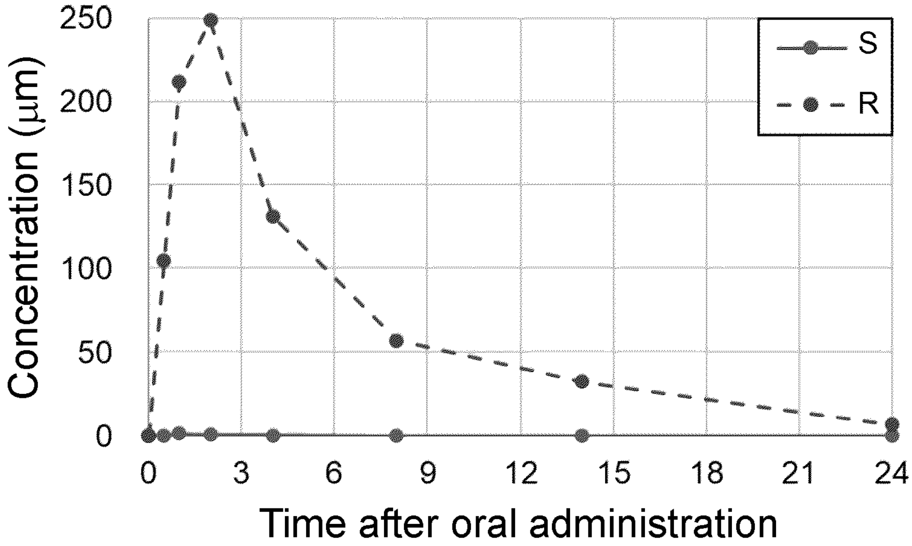
Concentration (μm)
250
200
150
100
50
0
0
3
6
9
12
15
18
21
24
Time after oral administration
S
R

[FIG.11 ]

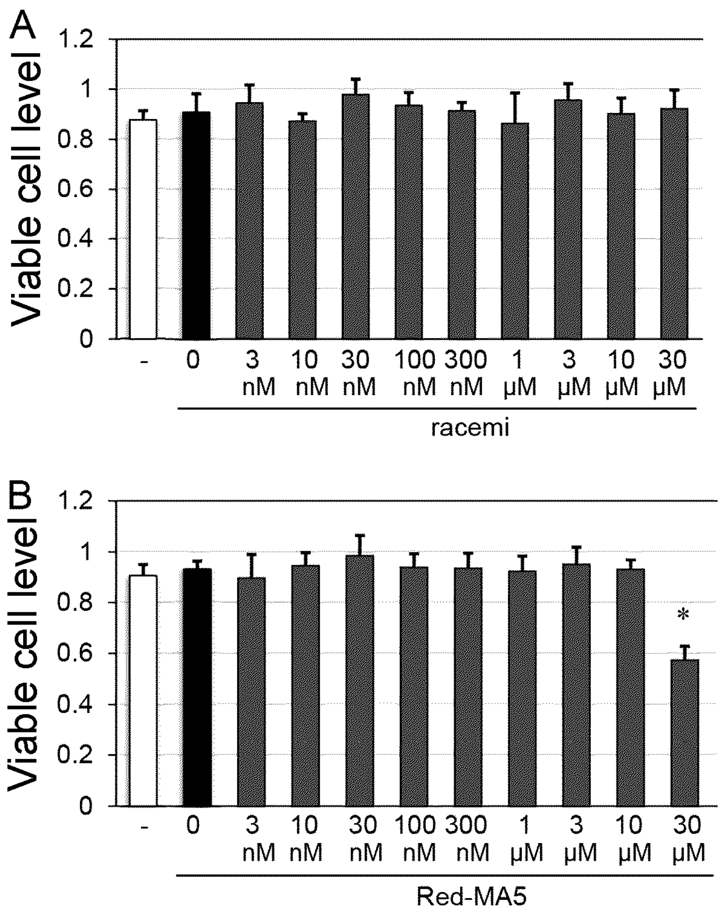
A
Viable cell level
1.2
1
0.8
0.6
0.4
0.2
0
-
0
3 nM
10 nM
30 nM
100 nM
300 nM
1 μM
3 μM
10 μM
30 μM
racemi
B
Viable cell level
*
Red-MA5

[FIG.12-1 ]

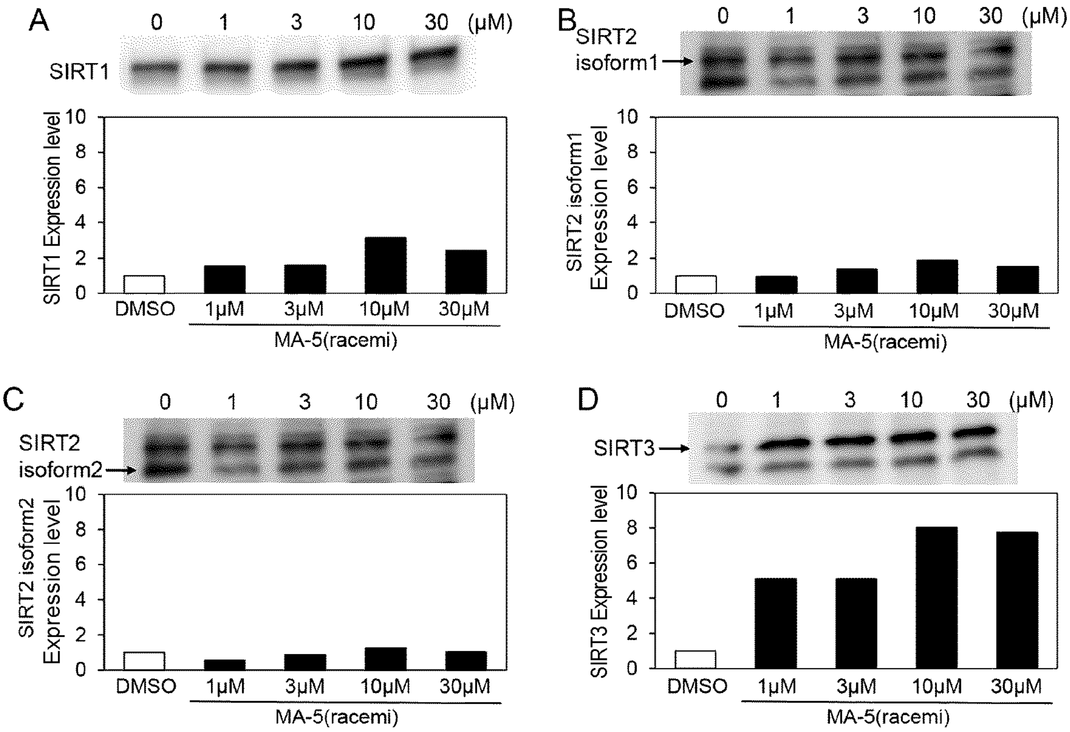


[FIG.12-2 ]

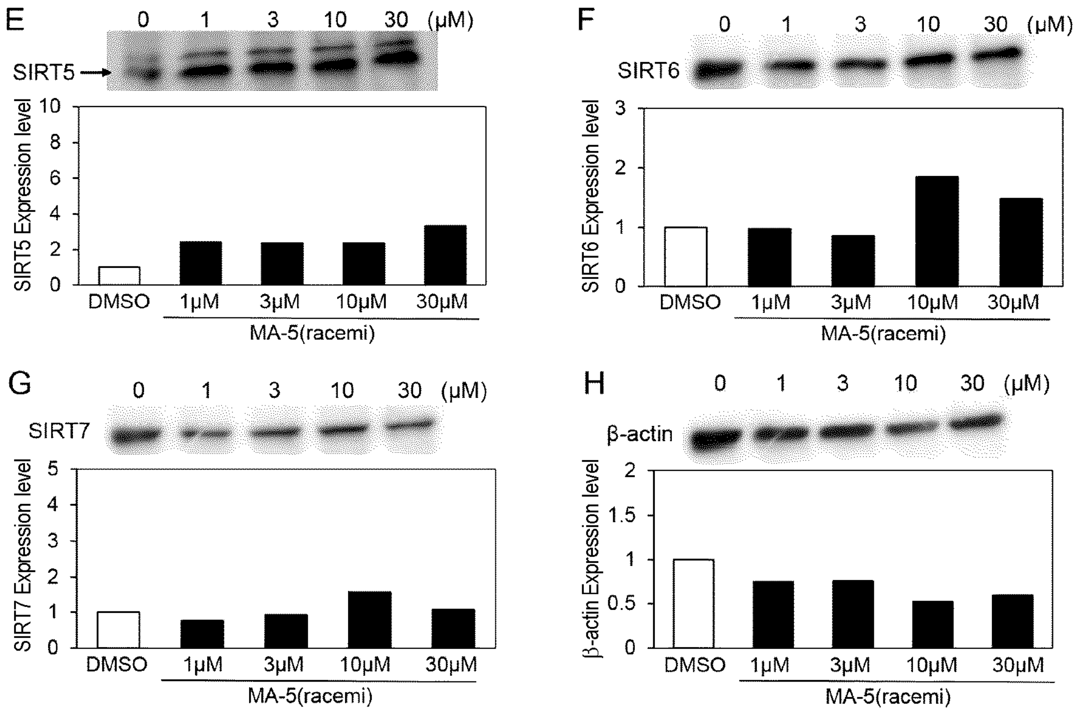

[FIG.13-1 ]

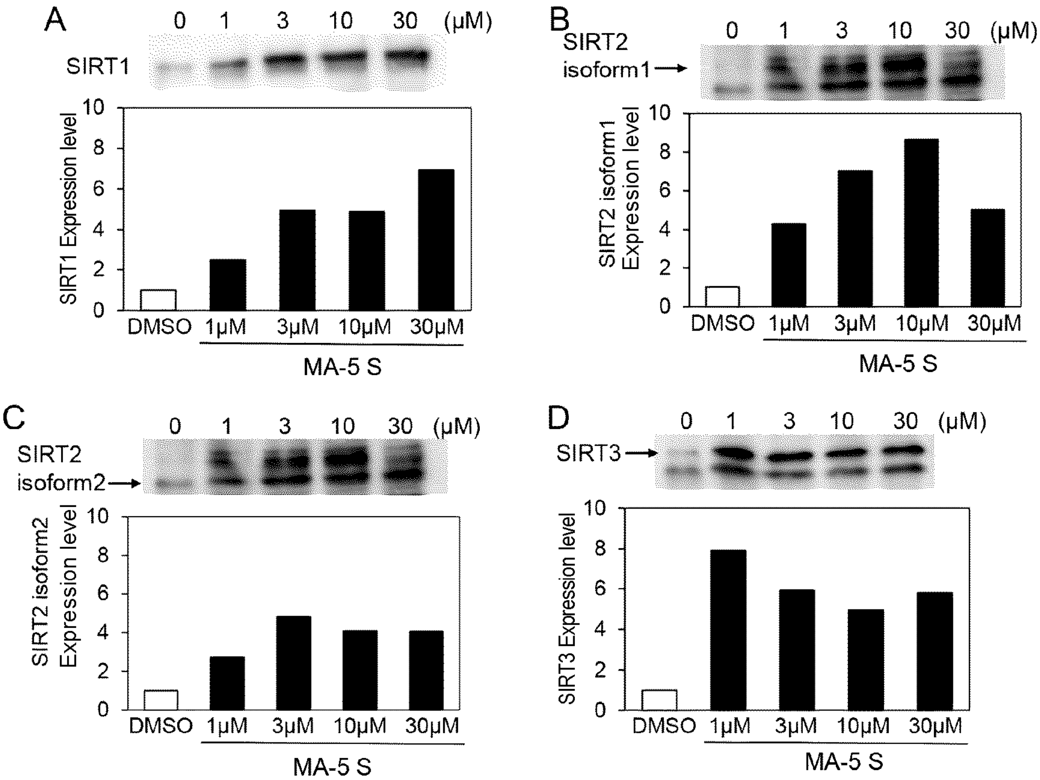


[FIG.13-2 ]

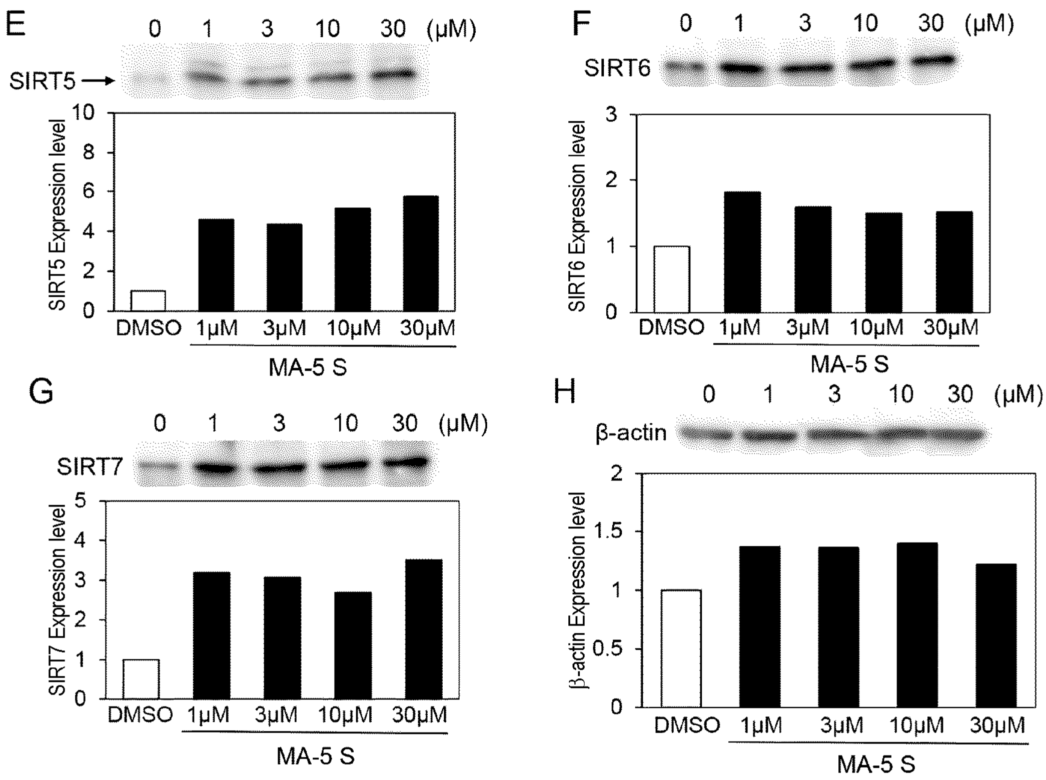

[FIG.14-1 ]

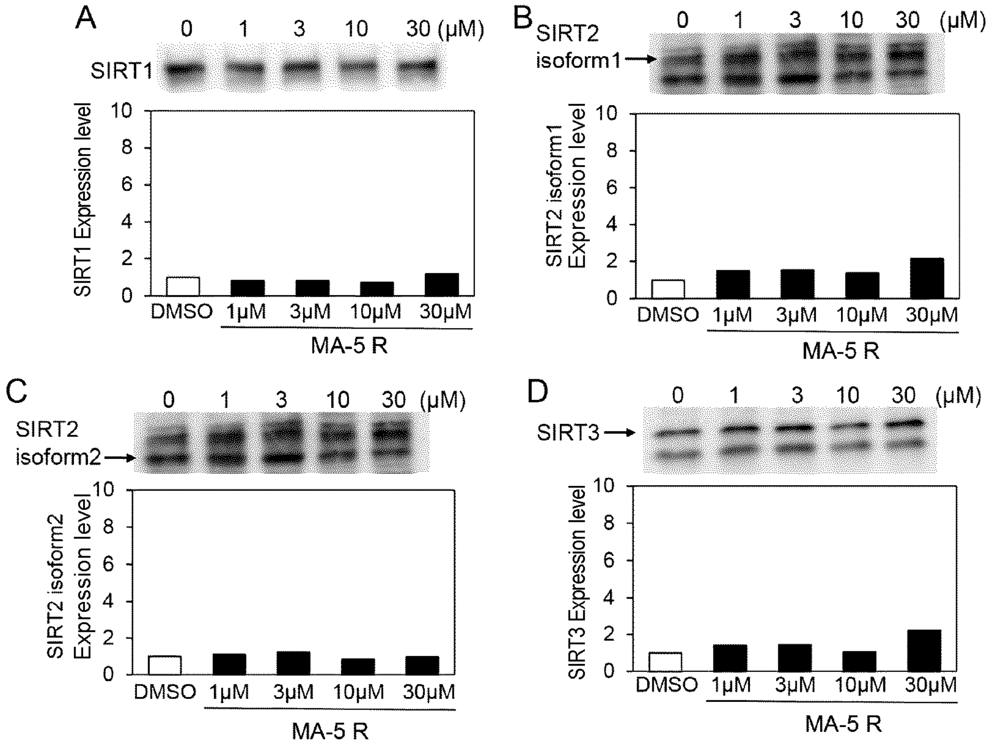


[FIG.14-2 ]

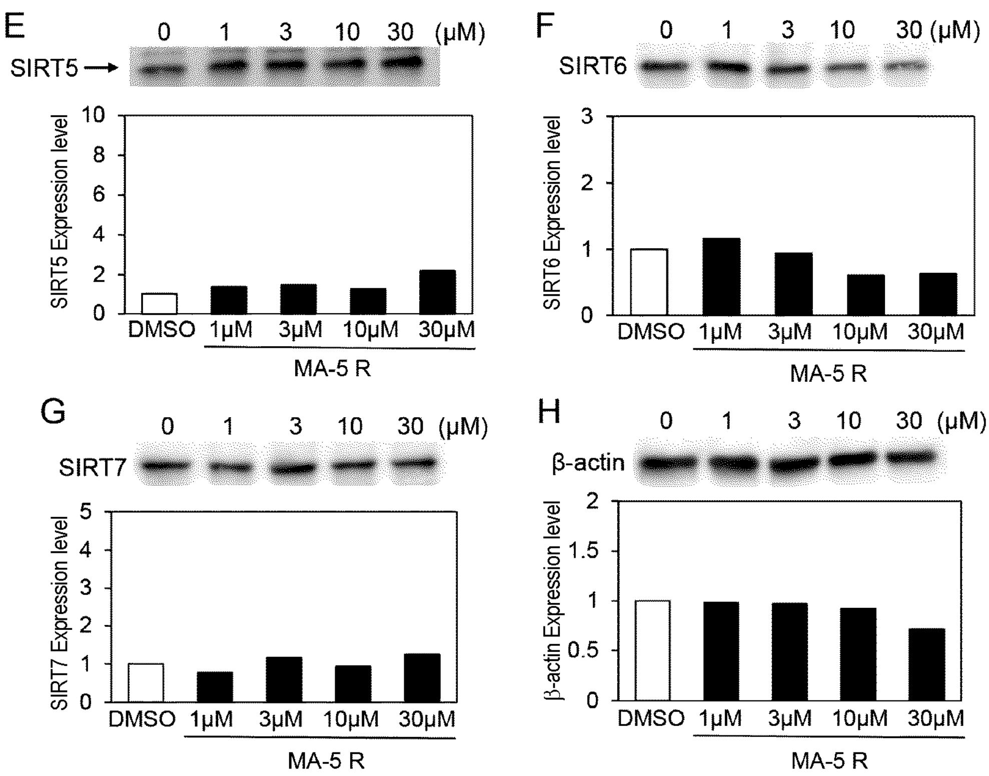

[FIG.15 ]

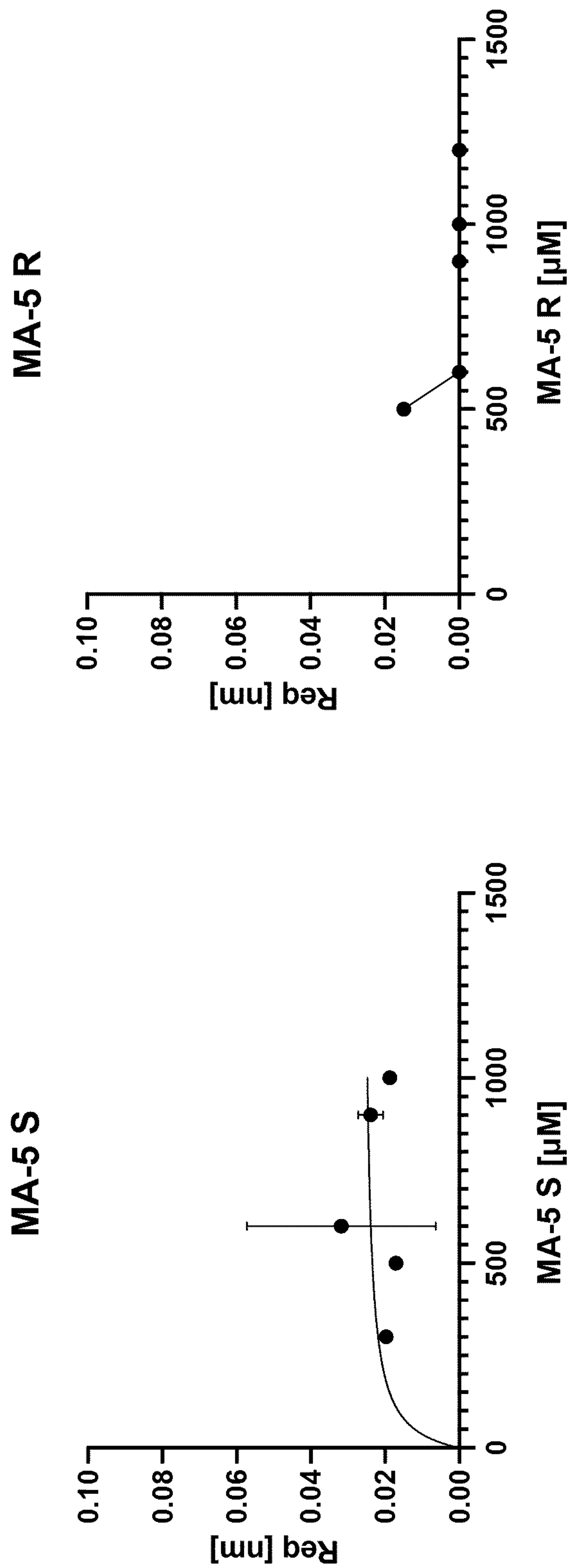
MA-5 R
Req [nm]
0.10
0.08
0.06
0.04
0.02
0.00
0
500
1000
1500
MA-5 R [µM]
MA-5 S
Req [nm]
0.10
0.08
0.06
0.04
0.02
0.00
0
500
1000
1500
MA-5 S [µM]

[FIG.16 ]

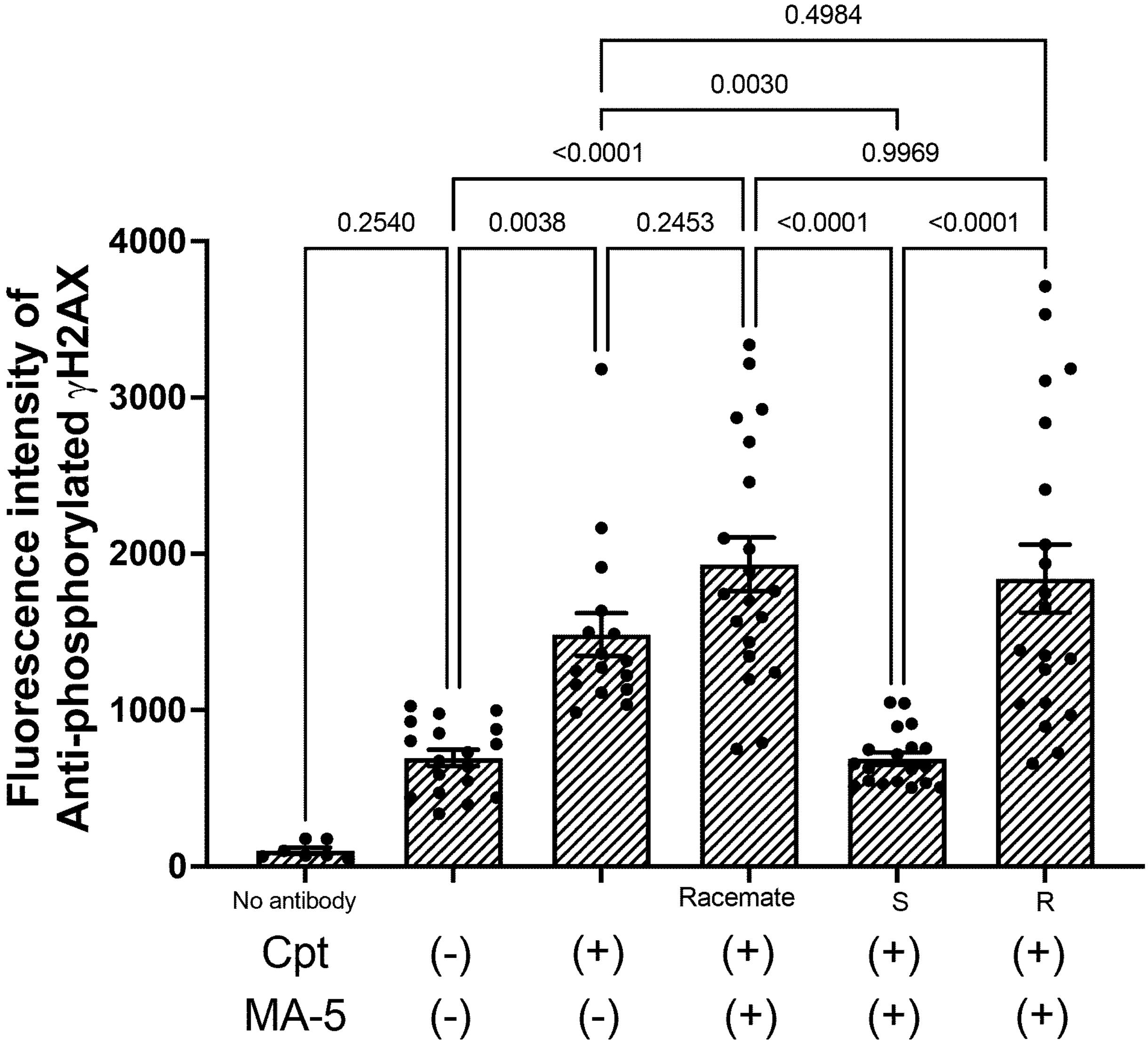
0.4984
0.0030
<0.0001
0.9969
0.2540
0.0038
0.2453
<0.0001
<0.0001
Fluorescence intensity of Anti-phosphorylated γH2AX
4000
3000
2000
1000
0
No antibody
Racemate
S
R
Cpt (-) (+) (+) (+) (+)
MA-5 (-) (-) (+) (+) (+)

[FIG.17 ]

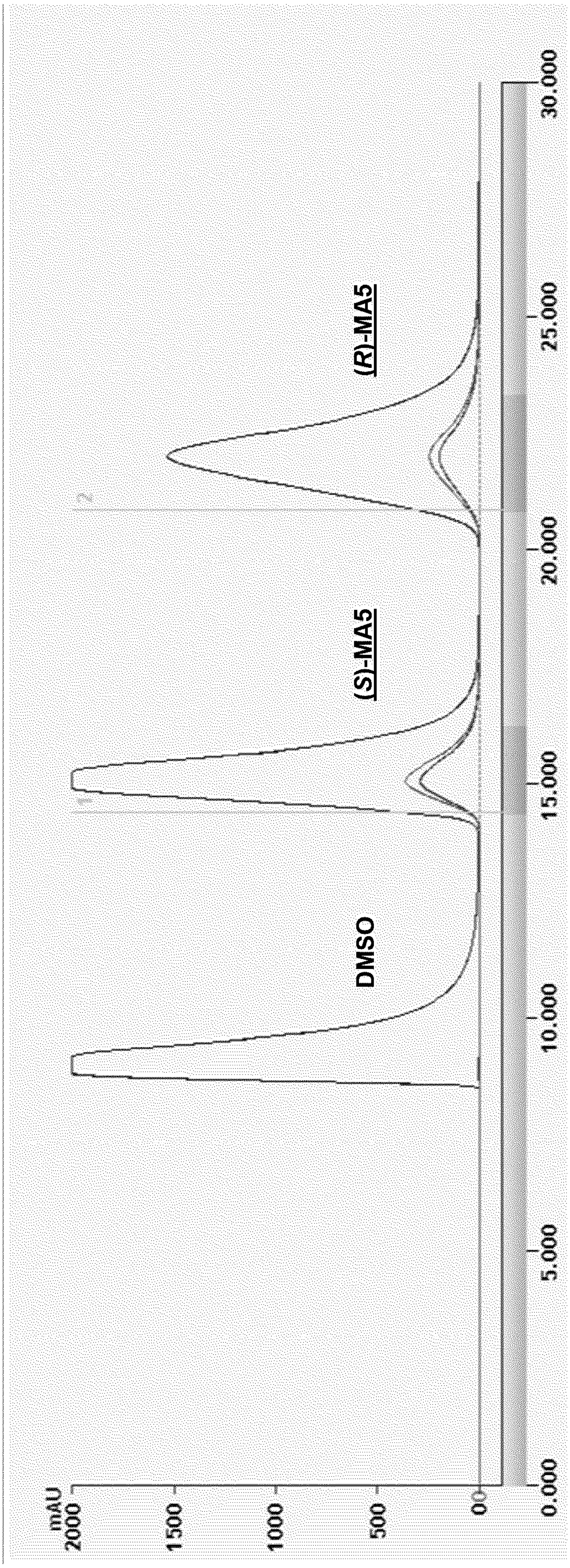
mAU
2000
1500
1000
500
0
0.000
5.000
10.000
15.000
20.000
25.000
30.000
DMSO
(S)-MA5
(R)-MA5
1
2

**INTERNATIONAL SEARCH REPORT**

International application No. **PCT/JP2024/036222**

**A. CLASSIFICATION OF SUBJECT MATTER**

***A61K 31/405***(2006.01)i; ***A61P 3/00***(2006.01)i; ***A61P 27/16***(2006.01)i; ***A61P 43/00***(2006.01)i
FI: A61K31/405; A61P3/00; A61P27/16; A61P43/00 105

According to International Patent Classification (IPC) or to both national classification and IPC

**B. FIELDS SEARCHED**

Minimum documentation searched (classification system followed by classification symbols)

A61K31/405; A61P3/00; A61P27/16; A61P43/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | WO 2023/013756 A1 (TOHOKU UNIVERSITY) 09 February 2023 (2023-02-09) paragraphs [0002], [0067]-[0069], [0073]-[0076], fig. 5, 7, 8 | 1, 3, 6-10, 13-14 |
| A | paragraphs [0002], [0067]-[0069], [0073]-[0076], fig. 5, 7, 8 | 2, 4-5, 11-12 |
| Y | JP 2018-76295 A (TAISHO PHARMACEUTICAL CO., LTD.) 17 May 2018 (2018-05-17) claims, examples | 1-2, 4, 6-9, 11, 13-14 |
| A | claims, examples | 3, 5, 10, 12 |
| Y | JP 2019-116453 A (TOHOKU UNIVERSITY) 18 July 2019 (2019-07-18) examples | 1-2, 4-9, 11-14 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 December 2024** | **10 December 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

INTERNATIONAL SEARCH REPORT

International application No.
PCT/JP2024/036222

C. DOCUMENTS CONSIDERED TO BE RELEVANT

| Category* | Citation of document, with indication, where appropriate, of the relevant passages | Relevant to claim No. |
|---|---|---|
| Y | 阿部高明, 世界初・日本発ミトコンドリア病治療薬ＭＡ－５のオールジャパン臨床治験, 2017年度「世界初・日本発ミトコンドリア病治療薬ＭＡ－５のオールジャパン臨床治験」成果報告書, 12 November 2018, (ABE, Takaaki, All-Japan clinical trial on the World's first Japanese-origin treatment drug for mitochondrial diseases MA-5), non-official translation (Final Report of 2017 All-Japan clinical trial on the World's first Japanese-origin treatment drug for mitochondrial diseases MA-5) "Pharmacology Testing" | 1-2, 4-9, 11-14 |
| P, X | US 2024/0270692 A1 (TOHOKU UNIVERSITY) 15 August 2024 (2024-08-15) claims, examples | 1-14 |
| A | 鈴木健弘ほか, ミトコンドリア病とその治療薬開発に向けて　ミトコンドリア創薬の基礎研究と臨床応用, 化学と生物, 2021, vol. 59, no. 7, pp. 339-345, (SUZUKI, Takehiro et al., Development of New Theraputics for Mitochondrial Disease: Bridging between Basic Researches and Clinical Implications of Mitochondrial Medicine, KAGAKU TO SEIBUTSU) entire text | 1-12 |

Form PCT/ISA/210 (second sheet) (July 2022)

**INTERNATIONAL SEARCH REPORT**
**Information on patent family members**

International application No.
**PCT/JP2024/036222**

| Patent document cited in search report | Publication date (day/month/year) | Patent family member(s) | Publication date (day/month/year) |
| --- | --- | --- | --- |
| WO 2023/013756 A1 | 09 February 2023 | (Family: none) | |
| JP 2018-76295 A | 17 May 2018 | (Family: none) | |
| JP 2019-116453 A | 18 July 2019 | US 2019/0224165 A1<br>examples | |
| US 2024/0270692 A1 | 15 August 2024 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2023036841 W
- WO 2014080640 A
- JP 2015189670 A
- JP 2019116453 A
- WO 2023013756 A


### Non-patent literature cited in the description


- *Nature*, 2018, vol. 563 (7731), 354-359
- Bulletin of the Institute of Advanced Biosciences. Tokai University, March 2017, vol. 1
- *Drug Metab. Pharmacokinet.*, 2010, vol. 25 (3), 274-282
- *Hum Mol Genet.*, 2019
- *FEBS Open Bio.*, 2022
- *Journal of Cellular Physiology*, 2010, vol. 224, 748-756
- *Molecular Biology of the Cell.*, 2011, vol. 22, 831-841
- *Cell*, 2009, vol. 139, 623-633
- *Nature*, 2018
- *Nat Rev Nephrol.*, 2017
- *Front Cell Dev Biol.*, 2021
- *Exp Cell Res.*, 11 July 2022, 113280
- *Hindawi Neural Plasticity*, vol. 2021